# EUROPÄISCHE PATENTSCHRIFT

(11) **EP 2 882 783 B1**
(45) Veröffentlichungstag und Bekanntmachung des Hinweises auf die Patenterteilung: **01.01.2020**
(21) Anmeldenummer: 13745037.5
(22) Anmeldetag: 30.07.2013
(51) Int. Cl.: C08F 6/06, C08F 6/10, C08F 6/16, C08F 26/10, C08L 39/06

(54) **VERFAHREN ZUR HERSTELLUNG WÄSSRIGER LÖSUNGEN VON VINYLLACTAM-POLYMEREN UND DEREN PULVER**
METHOD FOR PRODUCING WATERY SOLUTIONS OF VINYL LACTAM POLYMERS AND THEIR POWDER
PROCÉDÉ DE FABRICATION DE SOLUTIONS AQUEUSES À PARTIR DE POLYMÈRES DE LACTAME VINYLIQUE ET LEUR POUDRE

(30) Priorität: 08.08.2012 EP 12179719
(43) Veröffentlichungstag der Anmeldung: 17.06.2015
(73) Patentinhaber: BASF SE, 67056 Ludwigshafen am Rhein (DE)
(72) Erfinder: FILGES, Ulrich, 67433 Neustadt (DE); POTZOLLI, Bernd de, 67098 Bad Dürkheim (DE); WOLFF, Andy, 67459 Böhl-Iggelheim (DE)
(74) Vertreter: BASF IP Association
(86) Internationale Anmeldenummer: PCT/EP2013/065942
(87) Internationale Veröffentlichungsnummer: WO 2014/023602

(56) Entgegenhaltungen:
- WO-A1-2009/024457
- WO-A1-2010/072640
- DE-A1-102004 049 344
- DE-A1-102005 005 974
- US-A- 2 872 433

## Beschreibung

Die vorliegende Erfindung betrifft ein Verfahren zur Herstellung wässrigen Lösungen von Vinyllactam-Polymeren und durch Trocknung daraus erhältliche Feststoffe, insbesondere von Polyvinylpyrrolidon, wobei das Verfahren dadurch gekennzeichnet ist, dass eine oder mehrere Schwefelkomponenten, ausgewählt aus der Gruppe bestehend aus Schwefeldioxid, schwefliger Säure und einem oder mehreren Salzen der schwefligen Säure, zur Restmonomerabsenkung verwendet werden. Die Erfindung betrifft weiterhin nach dem Verfahren erhältliche Vinyllactam-Polymere, die eine gute Lagerstabilität aufweisen, sowie dessen Verwendung, Erzeugnisse und Zubereitungen, die diese Vinyllactam-Polymere enthalten oder unter deren Verwendung hergestellt wurden.

Die Herstellung von Polymerisaten des N-Vinylpyrrolidons durch radikalische Polymerisation ist bekannt. Der Mechanismus der Polymerisation unter verschiedenen Bedingungen ist beispiels-weise in Polymer Journal, 17, 143-152 (1985) beschrieben. In der Patentliteratur werden sowohl Polymerisationen in organischen Lösungsmittel wie z.B. in der US 4,053,696 in alkoholischer Lösung als auch in wässeriger Lösung wie z. B. in der US 2,335,454 beschrieben.

Die Polymerisation in organischen Lösungsmitteln, wie sie in der US 4,053,696 beschrieben ist, führt in der Regel zu Produkten, die nur einen relativ geringen Anteil an Verunreinigungen wie Ameisensäure enthalten. Dieses Verfahren hat aber den großen Nachteil, dass die Polymerisation zunächst in einem Lösungsmittel wie Isopropanol durchgeführt werden muss. Erst nach Beendigung der Polymerisation muss durch einen aufwändigen Destillationsprozess das Lösungsmittel z.B. das Isopropanol abdestilliert und durch Wasser ersetzt werden. Dabei fallen größere Mengen Lösungsmittel an, die entweder entsorgt oder destillativ gereinigt werden müssen. Dies führt zu einer langen Belegungszeit des Polymerisationsreaktors und einer ungünstigen Raum Zeit-Ausbeute.

Die Polymerisation von N-Vinylpyrrolidon in wässeriger Lösung wird dagegen meist in Gegenwart von Wasserstoffperoxid als Starter durchgeführt, wie es beispielsweise in der US 2,335,454 beschrieben ist. Das Molekulargewicht des Polyvinylpyrrolidons hängt dabei von der Wasserstoffperoxidkonzentration ab, wobei niedrige Molekulargewichte durch hohe Wasserstoffperoxidkonzentrationen entstehen und umgekehrt. Hohe Wasserstoffperoxidmengen begünstigen aber im wässrigen System die Bildung von Ameisensäure; solchermaßen hergestellte Polymere weisen nach der Polymerisation bereits eine deutliche Gelbfärbung auf.

In DE 11 2005 002 719 wird ein Verfahren zur Herstellung von wässerigen Polyvinylpyrrolidon-Polymer-Lösungen beschrieben. Weiterhin wird ein Verfahren zur Handhabung von Vinylpyrrolidon-Polymer-Lösungen offenbart. Mit diesem Verfahren werden Polymerisate mit niedriger HAZEN-Farbzahl (die Hazen-Farbzahl entspricht der "Kobalt-Platin-Farbzahl" und ist dem Fachmann als solches bekannt, etwa aus der Industrienorm DIN ISO 6271-1) erreicht. Die nach diesem Verfahren hergestellten Polymerlösungen haben den großen Nachteil, dass sie einerseits einen hohen Ameisensäure Anteil aufweisen und die Produkte sich allmählich bei Lagerung verfärben. Die Ameisensäure entsteht während der Polymerisation in einer Nebenreaktion als unerwünschte Nebenkomponente. Das erhaltene Polymerisat ist für häufige Anwendungszwecke ungeeignet, insbesondere dann, wenn aus den Lösungen durch Sprühtrocknung PVP-Pulver hergestellt werden. Für die Verwendung insbesondere für Pharma- und Kosmetik-Produkte sind Grenzwerte für Ameisensäure zu beachten. So ist etwa in der Europäischen Pharmakopöe in der Monographie "Povidone" ein Grenzwert für Ameisensäure von maximal 0,5 Gewichtprozent vorgeschrieben, und auch in kosmetischen Formulierungen darf der Ameisensäureanteil als Konservierungsmittel einen Grenzwert von 0,5 Gewichtprozent nicht überschreiten. Wie erwähnt verfärben sich die zunächst farblosen Lösungen bei längerer Lagerung.

Diese Eigenschaft der Verfärbungen ist aber gerade für Kosmetikformulierungen unerwünscht, speziell wenn daraus transparente, farblose Haargele hergestellt werden sollen.

Bühler berichtet über Farbveränderung bei wässrigen PVP-Lösungen insbesondere nach Lagerung oder Erwärmen, etwa bei der Sterilisierung. Die entstehende gelbe bis braungelbe Färbung resultiert aus der Oxidation mittels Luftsauerstoff. Dies kann laut Bühler durch Zusatz von geeigneten Antioxidantien vermieden werden. Bühler nennt als solche Antioxidantien Cystein und Natriumsulfit (Volker Bühler, "Polyvinylpyrrolidone - Excipients for Pharmaceuticals", Springer, 2005, Seiten 34 und 35 zur Stabilität in festen und flüssigen Dosierformen).

Die schon aus der Polymerisation stammenden sowie direkt danach entstehenden Peroxide haben die nachteilige Wirkung, dass sie zumindest teilweise bereits bei Zugabe zum Polymer aufgebraucht werden und so den Schutz und die Dauer der Lagerzeit verringern. Zur Kompensation dieses Effekts müssen daher größere Mengen an Antioxidans eingesetzt werden.

Die Oxidationsempfindlichkeit von Polymeren wie PVP, die makroskopisch sicht- und messbaren Auswirkungen der Oxidation sowie vorgeschlagene Maßnahmen zur Eindämmung und Hemmung der Oxidation, ist in vielen Veröffentlichungen beschrieben worden (siehe beispiels-weise Bühler in oben aufgeführter Veröffentlichung; Kline in Modern Plastics, 1945, November, ab Seite 157; Reppe in der Monografie zu PVP, Verlag Chemie, Weinheim, 1954, Seite 24; EP-B 873 130; US 6,331,333; US 6,498,231; Staszewska in "Die Angewandte Makromolekulare Chemie", 1983, 118, Seiten 1 bis 17).

In US 2,821,519 wird ein Verfahren zur Stabilisierung von PVP mittels Zusatz von Hydrazin und dessen Derivaten beschrieben.

Hydrazine sind toxikologisch bedenklich und insbesondere in Polyvinylpyrrolidonen, N-Vinylpyrrolidon-Copolymeren und Polymeren von N-Vinylpyrrolidon-Derivaten unerwünscht.

In EP-B 1 083 884 wird ein Verfahren zur Stabilisierung von Polyvinylpyrrolidonen gegen Peroxid-Bildung beschrieben. Wässrige Lösungen der Polymeren werden mit sehr geringen Mengen von Schwermetallsalzen oder mit Peroxid-spaltenden Enzymen versetzt. Diese verbleiben im Produkt. Geeignete Schwermetalle sind Mangan, Zink, Kobalt und insbesondere Kupfer. Der Einsatz der vorgeschlagenen Schwermetalle ist jedoch wegen möglicher Akkumulierung im Körper von Nachteil. Der Einsatz von Enzymen ist aus Kosten- und Stabilitätsgründen nachteilig.

Aus GB 836,831 [dem GB-Äquivalent zur US 2872433 = D2 im vorliegenden EP-Prüfungsverfahren] ist ein Verfahren zur Stabilisierung von Polyvinylpyrrolidonen gegen Verfärbungen bekannt, bei dem Lösungen der Polymere mit Schwefeldioxid, schwefliger Säure oder Alkalimetallsulfiten behandelt werden. Offenbart wird der Zusatz der Schwefel-Verbindung zu Polymer-Lösungen, die durch Auflösen von trockenem Polymerpulver in Wasser gewonnen werden, durch Einmischen bei Raumtemperatur. Die Schwefel-Verbindung wird als Reduktionsmittel eingesetzt, das bei hohen Temperaturen, denen die Polymerlösung ausgesetzt wird wie beim Sterilisieren oder beim Trocknen der Polymerlösungen, vor Gelbfärbung Schutz bieten soll.

Aus DE 10 2005 005 974 [= D1 im vorliegenden EP-Prüfungsverfahren] ist bekannt, dass bei dem aus GB 836,831 bekannten Verfahren der Peroxid-Aufbau durch Lagerung sogar in stärkerem Maße auftritt als bei unbehandelten Polymeren. DE 10 2005 005 974 offenbart ein Verfahren, wobei die Polyvinylpyrrolidone zunächst mit Schwefeldioxid, schwefliger Säure oder deren Alkalimetallsalzen und anschließend mit einem Radikalfänger behandelt werden. Die Schwefel-haltigen Reagenzien werden nach Ende der Nachpolymerisation und nach einer eventuellen sauren Hydrolyse direkt vor der optionalen Trocknung zugesetzt, eingerührt und dienen als Reduktionsmittel. Zusätzlich müssen Antioxidantien eingesetzt werden, um eine Stabilisierung zu erreichen.

Zur Restmonomerabsenkung werden nach dem Stand der Technik nach der Polymerisation wiederholt kleinere Initiator-Portionen zur Polymerlösung zugegeben und nachpolymerisiert (DE 11 2005 002 719). Diese beschriebene Nachbehandlung der DE 11 2005 002 719 mit erneuter Initiator Zugabe führt aber zu erhöhten und unerwünschten Formiat-Gehalten.

Die Restmonomerabsenkung durch Zusatz von organischen und anorganischen Säuren ist aus WO 93/16114 A1 bekannt: Offenbart wird die Absenkung auf pH-Werte kleiner 5 zur sauren Hydrolyse von Vinyllactamen zu freiem Lactam, wie Vinylpyrrolidon zu 2-Pyrrolidon.

DE 10 2004 049344 A1 [= D3 vorliegenden EP-Prüfungsverfahren] offenbart ein Verfahren zur Stabilisierung von Vinyllactam-Polymeren, insbesondere PVP, durch Zusatz kleiner Mengen von Wasserstoffperoxid. Ein Verfahren zur Restmonomerabsenkung und Stabilisierung unter Verwendung von solchen Schwefelverbindungen wie in der vorliegenden Offenbarung ist dort nicht beschrieben.

WO 2009/02447 A1 [= D4 vorliegenden EP-Prüfungsverfahren] offenbart ein Verfahren zur Herstellung von vernetzten Acrylsäurepolymeren, wobei wenigstens zwei verschiedene Radikalinitiatoren bei wenigstens zwei verschiedenen Polymerisationstemperaturen im Verfahren eingesetzt werden. Radikalisch polymerisierte, lineare (d.h. unvernetzte) lösliche Vinyllactam-Polymere wie in der vorliegenden Erfindung werden in WO 2009/02447 A1 nicht offenbart. Ein Verfahren zur Restmonomerabsenkung und Stabilisierung unter Verwendung von solchen Schwefelverbindungen wie in der vorliegenden Offenbarung ist dort ebenfalls nicht beschrieben.

WO 2010/072640 A1 [= D5 im vorliegenden EP-Prüfungsverfahren] offenbart die Behandlung von "PVPP", also "Polyvinlypyrrolidon-Popcorn-Polymer" und nicht radikalisch mittels Radikalinitiator polymerisierten Vinyllactam-Polymeren wie in der vorliegenden Anmeldung. Ein Popcorn-Polymer wird zwar auch radikalisch aber ohne Zusatz von Radikalinitiator hergestellt. Obwohl nur geringe Vernetzermengen von meist nicht mehr als 2 Gewichtsprozent vorhanden sind, sind Popcornpolymere hochverzweigt/vernetzt/verschlauft - die genaue Struktur ist bis heute nicht exakt nachgewiesen worden - und in allen Lösemitteln vollständig unlöslich, während lineares, radikalisch mit Radikalinitiatoren polymerisiertes "PVP" und Vinyllactam-Polymere in sehr vielen organischen wie wässrigen Lösemitteln vollständig löslich sind. Ein Verfahren zur Restmonomerabsenkung und Stabilisierung unter Verwendung von solchen Schwefelverbindungen wie in der vorliegenden Offenbarung ist dort ebenfalls nicht beschrieben.

Die Aufgabe der vorliegenden Erfindung war die Bereitstellung eines einfachen Verfahrens zur Herstellung von wässrigen PVP-Lösungen mit niedrigem Ameisensäure- bzw. Formiat-Gehalt sowie die Vermeidung von unerwünschten, beispielsweise toxikologisch bedenklichen Zusätzen wie Metallen, Enzymen oder Antioxidantien bei gleichzeitig hoher Farbstabilität während der Lagerung.

Diese Aufgabe wurde durch ein Verfahren zur Herstellung eines Polymers umfassend die folgenden Verfahrensschritte gelöst (allgemeine Ausführungsform A 1):
a) Polymerisation von Monomeren mittels freier radikalischer Polymerisation in einer Flüssigkeit,
b) optionales Verwenden einer Base als pH-Regulator während dieser Polymerisation,
c) optionale Nachpolymerisation,
d) optionales Reinigen mittels Strippung mit Gas, thermische Destillation und/oder Wasserdampfdestillation,
e) behandeln des Polymeren mit einer Schwefelkomponente, ausgewählt aus der Gruppe bestehend aus schwefliger Säure, Schwefeldioxid und einem oder mehreren Salzen der schwefligen Säure, wobei der pH-Wert einer wasserhaltigen Phase, mit dem das Polymer bei der Behandlung mit der Schwefelkomponente in Kontakt kommt, einen Wert von weniger als 6 aufweist, und In-Kontakt-halten des Polymers mit der wasserhaltigen Phase enthaltend die Schwefelkomponente bei diesem pH-Wert für einen Zeitraum zwischen 10 Minuten und 8 Stunden, und anschließend optionale Wiederholung von Schritt d),
f) optionale Zugabe einer Base zur Einstellung eines gewünschten pH-Wertes,
g) optionale Reinigung etwa mittels Filterung, und
h) optionales Trocknen des Flüssigkeitshaltigen Polymers zu einem Feststoff.

Bevorzugt umfasst der Verfahrensschritt e) das Behandeln des Polymeren mit einer Schwefelkomponente, ausgewählt aus der Gruppe bestehend aus schwefliger Säure, Schwefeldioxid und einem oder mehreren Salzen der schwefligen Säure, so dass der pH-Wert einer wasserhaltigen Phase, mit dem das Polymer bei der Behandlung in Kontakt kommt, einen Wert von weniger als 6, bevorzugt weniger als 5,5, besonders bevorzugt weniger als 5, ganz besonders bevorzugt weniger als 4 aufweist, und In-Kontakt-halten des Polymers mit der wasserhaltigen Phase enthaltend die Schwefelkomponente bei diesem pH-Wert für einen Zeitraum zwischen 10 Minuten und 8 Stunden, bevorzugt mindestens 30 Minuten und maximal 4 Stunden gehalten wird, und die anschließende optionale Wiederholung von Schritt d).

Eine erfindungsgemäße Ausführungsform ist ein Verfahren zur Herstellung eines Vinyllactam-Polymers mit K-Werten von 10 bis 150 umfassend die folgenden Verfahrensschritte a) bis h) (allgemeine Ausführungsform A 2):
a) Polymerisation von einem oder mehreren N-Vinyllactamen und optional weiteren Monomeren mittels freier radikalischer Polymerisation mit einem Radikal-bildenden Initiator in einer wässrigen Flüssigkeit, wobei das Polymerisationsverfahren als Batch-Verfahren, als-Semi-Batch-Verfahren oder kontinuierlich durchgeführt wird,
b) Verwendung wenigstens einer Base um den pH-Wert während der Polymerisation in einem Bereich von 5 bis 11 zu halten;
c) optionale Nachpolymerisation, wobei weiterer Initiator zugegeben werden kann;
d) optionale Reinigung mittels Strippung mit Gas, thermische Destillation und/oder Wasserdampfdestillation;
e) Behandeln des Vinyllactam-Polymeren mit einer Schwefelkomponente, wobei die Schwefelkomponente ausgewählt ist aus der Gruppe bestehend aus schwefliger Säure, Schwefeldioxid und einem oder mehreren Salzen der schwefligen Säure, wobei der pH-Wert einer wasserhaltigen Phase, mit dem das Polymer bei der Behandlung mit der Schwefelkomponente in Kontakt kommt, einen Wert von weniger als 5 aufweist, und In-Kontakt-halten des Polymers mit der wasserhaltigen Phase enthaltend die Schwefelkomponente bei diesem pH-Wert für einen Zeitraum zwischen 10 Minuten und 5 Stunden und anschließend optionale Wiederholung von Schritt d);
f) optionale Zugabe wenigstens einer Base zur Einstellung eines gewünschten pH-Wertes im Bereich von 4 bis 9;
g) optionale Reinigung mittels Filterung;
h) optionales Trocknen zu einem freifließenden Pulver.

Eine weitere, bevorzugte Ausführungsform 1 ist ein Verfahren zur Herstellung eines Vinyllactam-Polymers, bevorzugt eines Vinylpyrrolidon-Polymers, besonders bevorzugt Polyvinylpyrrolidon, mit K-Werten von 10 bis 150, bevorzugt 15 bis 130, besonders bevorzugt von 20 bis 95, ganz besonders bevorzugt von 20 bis 50 und insbesondere von 25 bis 35, umfassend die Verfahrensschritte a) bis h):
a) Polymerisation von einem oder mehreren N-Vinyllactamen, bevorzugt wenigstens Vinylpyrrolidon, und optional weiteren Monomeren, besonders bevorzugt nur Vinylpyrrolidon als Monomer, mittels freier radikalischer Polymerisation mit einem Radikal-bildenden Initiator, bevorzugt Wasserstoffperoxid mit einem Kupfer-Salz zur Steuerung der Radikalbildung, in einer wässrigen Flüssigkeit, bevorzugt in Wasser, wobei das Polymerisationsverfahren bevorzugt als Batch-Verfahren durchgeführt wird,
b) Verwendung wenigstens einer Base, bevorzugt ausgewählt aus der Gruppe bestehend aus Ammoniak, Ammoniumcarbonat, Ammoniumhydrogencarbonat, Triethanolamin, Diethanolamin, Monoethanolamin und Triethylamin, besonders bevorzugt ausgewählt aus der Gruppe bestehend aus Ammoniak, Ammoniumcarbonat und Ammoniumhydrogencarbonat, um den pH-Wert während der Polymerisation in einem Bereich von 5 bis 11, bevorzugt 5,5 bis 10,5, besonders bevorzugt 6 bis 9,5, und ganz besonders bevorzugt von 6,5 bis 9 zu halten, wie beispielsweise bei 7, 7,5, 8 oder 8,5;
c) optionale Nachpolymerisation, wobei weiterer Initiator, bevorzugt Wasserstoffperoxid als Radikalbildner mit einem Kupfer-Salz zur Steuerung der Radikalbildung, zugegeben werden kann, bevorzugt jedoch kein weiterer Initiator zugesetzt wird;
d) Reinigung mittels Strippung mit Gas, thermische Destillation und/oder Wasserdampfdestillation, bevorzugt durch Wasserdampfdestillation wenn die Flüssigkeit Wasser oder ganz überwiegend Wasser ist oder durch thermische Destillation und anschließende Wasserdampfdestillation, wenn die Flüssigkeit ein Gemisch aus Wasser und größeren Mengen organischen Lösemittels oder im Wesentlichen organisches Lösemittel ist;
e) Behandeln des Polymeren mit Schwefelkomponente, wobei die Schwefelkomponente ausgewählt ist aus der Gruppe bestehend aus schwefliger Säure, Schwefeldioxid und einem oder mehreren Salzen der schwefligen Säure, so dass der pH-Wert einer wasserhaltigen Phase, mit dem das Polymer bei der Behandlung in Kontakt kommt, einen Wert von weniger als 6, bevorzugt weniger als 5, besonders bevorzugt weniger als 4, aufweist, und In-Kontakt-halten des Polymers mit der wasserhaltigen Phase enthaltend die Schwefelkomponente bei diesem pH-Wert für einen Zeitraum zwischen 10 Minuten und 5 Stunden, bevorzugt mindestens 30 Minuten und maximal 2 Stunden gehalten wird, und anschließend optionale Wiederholung von Schritt d), bevorzugt unter Wiederholung von Schritt d),
f) optionale Zugabe wenigstens einer Base, bevorzugt ausgewählt aus der Gruppe bestehend aus Ammoniak, Ammonium(hydrogen)carbonat, Triethanolamin, Diethanolamin, Monoethanolamin und Triethylamin, bevorzugt ausgewählt aus Ammoniak, Triethanolamin und Triethylamin, zur Einstellung eines gewünschten pH-Wertes im Bereich von 4 bis 9, bevorzugt 5 bis 8 wie beispielsweise 4,5, 5, 5,5, 6, 6,5, 7, 7,5, 8 oder 8,5;
g) optionale Reinigung mittels Filterung, bevorzugt mittels eines Gewebefilters,
h) optionales Trocknen, bevorzugt mittels eines Sprühtrocknungsverfahrens, des Flüssigkeits-haltigen-Polymers zu einem Feststoff, bevorzugt einem freifließenden Pulver.

In einer weiteren bevorzugten Ausführungsform 2 wird in Ausführungsform 1) der Schritt f) durchgeführt, bevorzugt unter Verwendung von Ammoniak als Base.

In einer weiteren bevorzugten Ausführungsform 3 wird in Ausführungsform 1) der Schritt g) durchgeführt.

In einer weiteren bevorzugten Ausführungsform 4 wird in Ausführungsform 1) der Schritt h) durchgeführt, bevorzugt unter Einsatz eines Sprühtrocknungsverfahrens, und ein trockenes Polymerpulver erhalten.

In einer weiteren bevorzugten Ausführungsform 5 wird in Ausführungsform 1) der Schritt c) nicht ausgeführt.

In einer weiteren, besonders bevorzugten Ausführungsform 6 werden in Ausführungsform 1) die Schritte f), g) und h) durchgeführt und Schritt c) nicht durchgeführt, bevorzugt mit Ammoniak als Base im Schritt f), bevorzugt einem mechanischen Filter im Schritt g) und bevorzugt einem Sprühtrocknungsverfahren in Schritt h).

In einer weiteren, besonders bevorzugten Ausführungsform 7) werden in Ausführungsform 1) die Schritte f) g) und h) durchgeführt, Schritt c) nicht durchgeführt und wird nach Schritt e) ein weiterer Reinigungsschritt d) durchgeführt, bevorzugt mittels Wasserdampfdestillation in den Schritten d), bevorzugt mit Ammoniak als Base im Schritt f), bevorzugt einem mechanischen Filter im Schritt g) und bevorzugt mit einem Sprühtrocknungsverfahren in Schritt h).

In weiteren, ganz besonders bevorzugten Ausführungsformen 8 bis 14 werden die Ausführungsformen 1 bis 7 jeweils durchgeführt bei Verwendung von Ammoniumhydrogencarbonat als Base im Schritt b), die bevorzugt nur zu Beginn der Polymerisationsreaktion zugegeben wird.

In weiteren, ganz besonders bevorzugten Ausführungsformen 15 bis 21 werden die Ausführungsformen 1 bis 7 jeweils durchgeführt bei Verwendung von Ammoniak als Base im Schritt b), die bevorzugt nur zu Beginn der Polymerisationsreaktion zugegeben wird.

In weiteren, ganz besonders bevorzugten Ausführungsformen 22 bis 42 werden die Ausführungsformen 1 bis 21 jeweils durchgeführt bei Verwendung von Schwefeldioxid in wässriger Lösung als Schwefelkomponente.

In weiteren, insbesondere bevorzugten Ausführungsformen 43 bis 84 werden die Ausführungsformen 1 bis 42 jeweils so durchgeführt, dass das Verfahren nur aus den Verfahrensschritten a) bis h) einschließlich des wiederholten Schrittes d) nach Schritt e) besteht.

Ebenso gefunden wurde ein Polymer erhältlich nach dem erfindungsgemäßen Verfahren, bevorzugt nach einer der Ausführungsformen 1 bis 84, besonders bevorzugt nach einer der Ausführungsformen 22 bis 42 oder 64 bis 84, ganz besonders bevorzugt nach einer der Ausführungsformen 29 bis 42 oder 71 bis 84, insbesondere nach einer der Ausführungsformen 29 bis 35 oder 71 bis 77 wie 13, 14, 20, 21, 27, 28, 34, 35, 41, 42, 48, 49, 55, 56, 62, 63, 69, 70, 76, 77, 83 und 84, mit hoher Stabilität bezüglich physikalischer Parameter wie insbesondere Farbe, Geruch, Klarheit und Viskosität einer Lösung des Polymeren.

Ebenso gefunden wurde die Verwendung des erfindungsgemäß hergestellten Polymers und/oder eines Polymers erhältlich nach dem erfindungsgemäßen Verfahren als Hilfsmittel oder Wirkstoff im Bereich Kosmetik wie insbesondere Haarkosmetika wie Haargele, im Bereich Pharma, Tierfutter, Tiergesundheit, Technik wie insbesondere Membrane zur Trennung von Stoffen, Medizintechnik wie insbesondere zur Herstellung von Membranen zur Reinigung von Flüssigkeiten wie Blut und Wasser wie insbesondere Dialysemembranen, im Bereich Pflanzenschutz, Getränketechnologie oder Lebensmitteltechnologie.

Ebenfalls gefunden wurden Arzneimittel, die erfindungsgemäß hergestelltes Polymer und/oder Polymer erhältlich nach dem erfindungsgemäßen Verfahren enthalten.

Ebenfalls gefunden wurden Zusammensetzungen zur Haarpflege und -festigung, bevorzugt Haargele, insbesondere farblose und klare Haargele, die erfindungsgemäß hergestelltes Polymer und/oder Polymer erhältlich nach dem erfindungsgemäßen Verfahren enthalten.

Ebenfalls gefunden wurden Membrane, bevorzugt zur Reinigung von Flüssigkeiten wie Blut und Wasser, insbesondere Dialysemembrane, die erfindungsgemäß hergestelltes Polymer und/oder Polymer erhältlich nach dem erfindungsgemäßen Verfahren enthalten.

Als "Schwefelkomponente" wird im Rahmen der vorliegenden Erfindung eine Substanz ausgewählt aus der Gruppe bestehend aus schwefliger Säure, Schwefeldioxid und einem oder mehreren Salzen der schwefligen Säure bezeichnet. "Eine" Schwefelkomponente bezeichnet dabei eine einzelne Verbindung oder mehrere Verbindungen ausgewählt aus der bezeichneten Gruppe, wenn sich nicht eindeutig aus der Beschreibung ergibt, dass nur "eine einzige" Schwefelkomponente gemeint ist.

Eine "wässrige Lösung von Schwefeldioxid" wird meist - jedoch chemisch falsch - als "schweflige Säure" bezeichnet. Die Bezeichnungen "Schwefeldioxid in wässriger Lösung", "eine wässrige Lösung von Schwefeldioxid" und "schweflige Säure" sind demnach Bezeichnungen für ein und dieselbe Substanz.

Mittels des erfindungsgemäßen Verfahrens können prinzipiell alle Homo- und Copolymere von N-Vinyllactamen erhalten werden.

Der Begriff "Polymer" umfasst beispielsweise lineare, wasserlöslich verzweigte oder wasserun-löslich verzweigte Polymere. Der Begriff "wasserunlöslich verzweigtes Polymer" umfasst auch die sogenannten Popcorn-Polymere, die im Englischen als "proliferous polymers" oder wie bei Polyvinylpyrrolidon als PVPP bezeichnet werden.

"Verzweigt", "verzweigend", "vernetzt", "vernetzend" wird im Rahmen dieser Erfindung austauschbar verwendet und meint Polymer, das mindestens eine Verzweigungsstelle aufweist. "Polymer" umfasst auch die Copolymere, Graft-Homo- oder Graft-co-Polymere, die jeweils als lineare oder löslich-vernetzte, insbesondere wasserlöslich vernetzte oder unlöslich-vernetzte, insbesondere wasserlöslich-vernetzte, Polymere vorliegen können.

"Polymer" kann in jedweder Form vorliegen, etwa als Di- oder Multi-Blockpolymere, wie auch in Stern-, Bürsten- oder hyperverzweigter Form oder als Dendrimer vorliegen.

Bevorzugte Polymere sind lineare, unvernetzte Polymere, besonders bevorzugt wasserlösliche, lineare, unvernetzte Polymere.

Erfindungsgemäße Polymere enthalten ein oder mehrere Monomere a), gegebenenfalls ein oder mehrere Monomere b) sowie gegebenenfalls ein oder mehrere vernetzende Monomere c), das heisst sie sind durch Polymerisation der genannten Monomeren erhalten worden und können noch Restmengen der Monomeren enthalten.

Monomere a) sind ausgewählt aus:
N-Vinyllactame wie N-Vinylpyrrolidon, N-Vinylpiperidon, N-Vinylcaprolactam, deren mit C1 bis C8-Alkyl-Gruppen-substiutierten Derivate wie 3-Methyl-, 4-Methyl- und 5-Methyl-N-Vinylpyrrolidon, N-Vinylamide wie N-Vinylformamid und dessen nach der Polymerisation durch Hydrolyse erhältliches N-Vinylamin, N-Vinyl-N-methylacetamid, Amine wie N-Vinyl- oder Allyl-substituierte heterocyclische Verbindungen, bevorzugt N-Vinylpyridin, N-Allylpyridin, N-Vinylimidazole, die auch in 2-, 4- oder 5-Position mit C1-C4-Alkyl, insbesondere Methyl- oder Phenyl-Resten substituiert sein können, wie 1-Vinylimidazol, 1-Vinyl-2-methylvinylimidazol sowie deren quaternisierte Analoga wie 3-Methyl-1-vinylimidazoliumchlorid, 3-Methyl-1-vinylimidazoliummethylsulfat, N-C1-bis C24-Alkyl-substituierte Diallylamine und deren quaternisierte Analoga wie Diallylammoniumchlorid und Diallyldimethylammoniumchlorid

Bevorzugte Monomere a) sind Vinyllactame wie N-Vinylpyrrolidon, 3-Methyl-N-vinylpyrrolidon, 4-Methyl-N-vinylpyrrolidon, 5-Methyl-N-vinylpyrrolidon, N-Vinylpiperidon und N-Vinylcaprolactam, Vinylacetat sowie der durch Hydrolyse nach der Polymerisation erhältliche Vinylalkohol, Vinylamide wie Vinylformamid sowie das durch Hydrolyse nach der Polymerisation erhältliche Vinylamin, N-Vinylimidazol, 1-Vinyl-3-methylimidazolium Chlorid, 1-Vinyl-3-methylimidazolium Sulfat, und Vinylmethylamid sowie deren Derivate.

Ganz besonders bevorzugte Monomere a) sind N-Vinylpyrrolidon, N-Vinylcaprolactam, Vinylacetat, Vinylformamid sowie das durch Hydrolyse nach der Polymerisation erhältliche Vinylamin sowie N-Vinylimidazol.

Erfindungsgemäße Polymere enthalten immer mindestens ein Vinyllactam-Monomer ausgewählt aus der Gruppe der Monomere a).

Erfindungsgemäße Polymere können Homopolymere als auch Copolymere aus zwei oder mehreren der Monomere a) sein, beispielsweise Copolymere aus N-Vinylpyrrolidon und N-Vinylimidazol, Copolymere aus N-Vinylpyrrolidon und N-Vinylformamid, Copolymere aus N-Vinylpyrrolidon und N-Vinylcaprolactam, Copolymere aus N-Vinylpyrrolidon, N-Vinylcaprolactam und N-Vinylimidazol oder Copolymere aus N-Vinylpyrrolidon und N-Vinylimidazol.

Als Monomere b) kommen alle Monomere in Betracht, die in WO 2010/072640 A1 als "Monomer b)" auf den Seite 6, ab Zeile 8, bis Seite 8, Zeile 17, genannt sind..

Bevorzugte Monomere b) sind Maleinsäure, Maleinsäureanhydrid, Isopropylmethacrylamid, Acrylamid, Methacrylamid, 2-Hydroxyethylacrylamid und 2-Hydroxyethylmethacrylamid, ferner, Vinylester aliphatischer C2-C18-Carbonsäuren wie Vinylacetat sowie der durch Hydrolyse nach der Polymerisation erhältliche Vinylalkohol, Vinylpropionat, Vinylbutyrat, Vinyllaurat, Vinylstearat, Vinylneodecanoat VEOVA 9 und VEOVA 10, ferner Dimethylaminoethyl(meth)acrylat und Dimethylaminoethyl(meth)acrylamid und deren quaternierte Analoga sowie Diallyldimethylam-moniumchlorid.

Ganz besonders bevorzugte Monomere b) sind Methacrylamid, Vinylacetat sowie der durch Hydrolyse nach der Polymerisation erhältliche Vinylalkohol, Vinylpropionat, Vinylneodecanoat VEOVA 9 und VEOVA 10, Dimethylaminoethyl(meth)acrylat und Dimethylaminoethyl(meth)acrylamid und deren quaternierte Analoga sowie Diallyldimethylammoniumchlorid.

Polymere, die Copolymere sind und Monomere b) enthalten, können eines oder mehrere der Monomere b) enthalten. Üblicherweise sind jedoch nicht mehr als fünf verschiedene Monomere b) in einem Copolymer enthalten.

Zu den bevorzugten Polymeren zählen des weiteren Copolymere, die ein oder mehrere Monomere a) und ein oder mehrere Monomere b) enthalten.

Vernetzende Monomere c) ("Vernetzer") sind Monomere mit zwei oder mehr radikalisch polymerisierbaren Gruppen. Geeignete vernetzende Monomere c) sind beispielsweise in WO2009/024457 auf Seite 7, Zeile 1, bis Seite 9, Zeile 2, beschrieben.

Besonders bevorzugt als vernetzende Monomere c) sind Pentaerythrittriallylether, Methylenbis-acrylamid, N,N'-Divinylethylenharnstoff, Divinylbenzol, Ethylen-bis-N-vinylpyrrolidon, 3-Vinyl-N-vinylpyrrolidon, 4-Vinyl-N-vinylpyrrolidon, 5-Vinyl-N-vinylpyrrolidon, Allyl(meth)acrylat, Triallylamin, Acrylsäureester von Glykol, Butandiol, Trimethylolpropan und Glycerin sowie Acrylsäureester von mit Ethylenoxid und/oder Epichlorhydrin umgesetztem Glykol, Butandiol, Trimethylolpropan und Glycerin. Ganz besonders bevorzugte Vernetzer sind Pentaerythrittriallylether, Methylenbisacrylamid, N,N'-Divinylethylenharnstoff, Triallylamin und Ethylen-bis-N-vinylpyrrolidon, für wasserunlöslich vernetzte Polymere inbesondere N,N'-Divinylethylenharnstoff, für wasserlöslich-vernetzte Polymere insbesondere Pentaerythrittriallylether und Triallylamin.

Die zur Polymerisation eingesetzten Monomere a), b) und c) können unabhängig voneinander ein einzelnes oder Mischungen mehrere Monomerer a), Monomerer b) und/oder Monomerer c) sein, wobei der gemeinsame Mengenanteil der Monomere a), b) oder c) den jeweils dafür genannten Mengenanteil für Monomer a), für Monomer b) beziehungsweise für Monomer c) am Polymer ergibt.

Die Gesamtmengen an Monomer(en) a) plus Monomer(en) b) plus Monomer(en) c) addieren sich immer zu 100 Gewichtprozent.

Die Mengenanteile in Gewichtprozent bezogen auf die Gesamtmasse des Polymeren betragen für die Monomere a) üblicherweise mindestens 20, bevorzugt mindestens 30, besonders bevorzugt mindestens 50, ganz besonders bevorzugt mindestens 60 Gewichtprozent und insbesondere bis zu 100 Gewichtprozent wie beispielsweise Homopolymere aus 100% eines Monomeren a).

Die Mengenanteile in Gewichtprozent bezogen auf die Gesamtmasse des Polymeren betragen für die Monomere b) üblicherweise bis zu 80, bevorzugt bis zu 70, besonders bevorzugt bis zu 50, ganz besonders bevorzugt bis zu 40 und insbesondere weniger als 5 Gewichtprozent und sind beispielsweise gar nicht im Polymer vorhanden.

Wenn das Polymer ein vernetztes Polymer ist, betragen die Mengenanteile der vernetzenden Monomere c) in Gewichtprozent bezogen auf die Gesamtmasse des Polymers üblicherweise 0,001 bis 20, bevorzugt 0,01 bis 10, besonders bevorzugt 0,05 bis 5 und insbesondere 0,1 bis 3 Gewichtprozent wie 0,2, 0,3, 0,4, 0,5, 0,7, 1,0, 1,5, 2 oder 2, 5.

Wird vernetzendes Monomer c) eingesetzt, so reduzieren sich die oben angegebenen Gesamt-Mengenanteile von Monomer a) und die oben angegebenen Gesamt-Mengenanteile von gegebenenfalls eingesetztem Monomer b) am Gesamt-Polymerfestgehalt entsprechend um die eingesetzte Gesamtmenge an vernetzendem Monomer c).

Ein Vinyllactam-Polymer kann demnach ein Homo- oder Copolymer sein enthaltend N-Vinyllactame wie N-Vinylpyrrolidon (VP) oder deren an 3, 4- oder 5-Position Methyl-substituierten Derivate, N-Vinylpiperidon oder N-Vinylcaprolactam (VCap). Bevorzugt ist N-Vinylpyrrolidon, N-Vinylcaprolactam oder deren Mischung. Insbesondere bevorzugt ist N-Vinylpyrrolidon.

Bevorzugte Vinyllactam-Polymere sind Vinylpyrrolidon-Polymere wie Polyvinylpyrrolidone, Vinylpyrrolidon-Copolymere und Vinylpyrrolidon-Popcorn-Polymere.

Erfindungsgemäße Polymere enthalten dabei immer wenigstens ein N-Vinyllactam-Monomer, bevorzugt N-Vinylpyrrolidon und/oder N-Vinylcaprolactam, besonders bevorzugt N-Vinylpyrrolidon und ganz besonders bevorzugt nur N-Vinylpyrrolidon als Vinyllactam. Insbesondere bestehen erfindungsgemäße Polymer nur aus N-Vinylpyrrolidon als einzigem Monomer.

Bevorzugte Polyvinylpyrrolidone sind Polymere mit K-Werten von 1 bis 150, vorzugsweise K10 bis K120, beispielsweise K12, K15, K 17, K25, K30, K60, K85, K90, K95, K100, K115 oder K120. Besonders bevorzugte PVP-Homopolymere weisen einen K-Wert von 12 bis 95 und ins-besondere bevorzugt von 15 bis 40 auf wie insbesondere K 20, K 25, K 30 und K 35.

Bevorzugte Vinylpyrrolidon-Copolymere sind lineare, unvernetzte Copolymere mit N-Vinylcaprolactam (VCap), Vinylacetat (VAc), N-Vinylimidazol (VI) und/oder dessen Derivaten und/oder deren Mischungen.

Besonders bevorzugte Copolymere sind Copolymere aus N-Vinylpyrrolidon (VP) mit Vinylacetat mit einem Gewichtsverhältnis VP/VAc von 20:80 bis 80:20, beispielsweise 30:70, 50:50, 60:40, 70:30, mit K-Werten von 10 bis 150, vorzugsweise von 15 bis 80 und insbesondere von 20 bis 50, beispielsweise 25, 30, 35, 40 oder 45. Ganz besonders bevorzugte Copolymere aus N-Vinylpyrrolidon mit Vinylacetat weisen einen K-Wert von 25 bis 45 und ein Gewichtsverhältnis VP zu VAc von 55:45 bis 70:30 auf wie 60:40.

Ebenso bevorzugt sind Copolymere aus VP und VCap mit K-Werten von 10 bis 100, vorzugsweise von 12 bis 80 und insbesondere von 20 bis 70, beispielsweise 30, 40, 50, oder 60, sowie Gewichtsverhältnissen der Monomere VP zu VCap von 80:20 bis 20:80, bevorzugt von 70:30 bis 30:70, insbesondere bevorzugt von 60:40 bis 40:60 und beispielsweise auch 50:50.

Der K-Wert der Vinylpyrrolidon-Copolymere und der Polyvinylpyrrolidone (Fikentscher K-Wert; siehe etwa Bühler, "Polyvinylpyrrolidone - Excipient for Pharmaceuticals", Springer, 2005, Seite 40 bis 41) ist ein Maß für die Lösungsviskosität bei definierten Bedingungen. Damit ist er ein direktes Maß für die Molmasse. Verändert sich die Molmasse beispielsweise durch oxidative Prozesse (erkennbar etwa an einem Anstieg des Peroxidgehaltes und/oder Farbvertiefung/Gelbfärbung), führt dies zu Molmassenaufbau (führt zur K-Wert-Erhöhung) oder zu Molmassenabbau (führt zur K-Wert-Erniedrigung) und so zu einer Veränderung des K-Werts. Verändert sich die Molmasse, so verändert sich entsprechend auch die Lösungsviskosität einer Lösung mit einem definierten Festgehalt.

Die Herstellung von N-Vinyllactam-Polymeren durch radikalische Polymerisation ist an sich bekannt. Die radikalische Polymerisation mit vernetzten Monomeren c) liefert verzweigte oder vernetzte Polymere, die je nach dem Grad der Vernetzung wasserlöslich bis in wasserunlöslich und beispielsweise in Wasser gelbildend sind. Die nach der Popcorn-Polymerisation (im Englischen meist als "proliforous polymerisation" bezeichnet) hergestellten Polymere sind dagegen in Wasser und allen Lösemitteln üblicherweise unlöslich. Die Polymerisation ohne vernetzende Monomere c) liefert dagegen üblicherweise lineare, unvernetzte Polymere.

Die Monomeren können nach den üblichen Verfahrenstechniken radikalisch polymerisiert werden, etwa nach der sogenannten Batch-Polymerisation, bei der Monomer/-e in Lösemittel, etwa Wasser, vorlegt wird/werden und der Initiator, etwa Wasserstoffperoxid und ein Kupfer-(II)-salz als Katalysator, bei erhöhter Temperatur von 30 bis 150 ° C, bevorzugt von 40 bis 95 ° C zugegeben wird. Die Base, meist Ammoniak, wird meist ebenfalls mit dem (den) Monomer(en) vorgelegt, kann aber auch inkrementell oder kontinuierlich zudosiert werden. Das Reaktionsgemisch wird bevorzugt solange bei der Polymerisationstemperatur gerührt, bis die Umsetzung mehr als 99,5 Gew. % beträgt. Meist wird gegen Ende eine zusätzliche Menge an Initiator zugegeben und oft auch die Reaktionstemperatur erhöht. Diese Phase ab Zugabe zusätzlichen Initiators/Erhöhung der Reaktionstemperatur wird üblicherweise als "Nachpolymerisation" bezeichnet. Ist die Polymerisationstemperatur zu Beginn zu niedrig, so startet die Reaktion schlecht oder gar nicht. Bei zu hohen Temperaturen nimmt die Verfärbung der Polymeren zu.

Alternativ zur Batch-Polymerisation kann auch eine Semi-Batch-Polymerisation (deutsch auch als "Zulauffahrweise" bezeichnet) durchgeführt werden. Dabei wird ein Teil oder die Gesamtmenge an Monomer während der Polymerisation zudosiert. Üblicherweise jedoch wird ein meist kleinerer Teil der Monomere in der Reaktionsmischung vorgelegt, der verbleibende Teil über einen gewissen Zeitraum zudosiert. Die Dosierung des Initiators dauert dabei üblicherweise länger als die Dosierung der Monomere.

Möglich ist auch die Polymerisation als kontinuierliche Polymerisation. Die Monomere und der Initiator wie auch Lösemittel werden dabei kontinuierlich und üblicherweise parallel in ein Reaktionsgefäß, etwa eine sogenannte Rührkesselkaskade oder einen Rohrreaktor, eingebracht, während am "Ende" der Kaskade oder des Rohres gleichzeitig zu der Zugabemenge aus Monomer, Initiator und Lösemittel" am Anfang" eine entsprechende Menge an Reaktionsmischung entnommen wird.

Bevorzugt wird das vorliegende Verfahren in Rührkesseln nach der Batch- oder der Semi-Batch-Methode durchgeführt. In einer alternativen Ausführungsform wird das Verfahren bevorzugt als kontinuierliche Polymerisation in Rohrreaktoren im Mikromaßstab durchgeführt. Mikromaßstab bedeutet, dass die Innendurchmesser der einzelnen Rohrreaktoren kleiner sind als 2 Zentimeter, bevorzugt kleiner als 1 Zentimeter.

Besonders bevorzugt für das vorliegende Verfahren ist die Semi-Batch- und die Batch-Polymerisation, ganz besonders bevorzugt die Batch-Polymerisation, insbesondere in üblichen Rührkesseln.

Die Polymerisation findet nach den Verfahren im Stand der Technik in einer Flüssigkeit statt.

Unter "Flüssigkeit" werden im Rahmen der vorliegenden Offenbarung alle Substanzen verstanden, die einen Schmelzpunkt von weniger als 100 ° C aufweisen und daher wenigstens in einem Teilbereich des Temperaturbereichs von Null bis 100° C bei Normaldruck flüssig vorliegen oder die zumindest in einem solchen Teilbereich durch Druckerhöhung über Normaldruck (Umgebungsdruck) flüssig werden.

Flüssigkeiten in diesem Sinne sind daher organische und anorganische Substanzen wie organische Lösemittel, anorganische und organische Salze sowie Gase. Als Flüssigkeit kann ebenso eine Mischung aus zwei oder mehreren unterschiedlichen Flüssigkeiten verwendet werden. Unter Flüssigkeit ist eine solche zu verstehen, die bei der radikalischen Polymerisation der erfindungsgemäßen Monomere inert oder im Wesentlichen inert ist. Die Flüssigkeit kann Lösungsmittel oder Dispersionsmittel für das Polymer sein. "im Wesentlichen inert" bedeutet dabei, dass die aus einer Reaktion mit dem Lösemittel entstandenen Nebenkomponenten weniger als 1000 ppm, bevorzugt weniger als 500 ppm und geringer am Polymerfestgehalt betragen.

Typische Vertreter der organischen Lösemittel sind etwa C1- bis C8-Alkohole wie Methanol, Ethanol, n-Propanol, iso-Propanol, n-Butanol und Strukturisomere, Glykol, Glyzerin, Diethylether. Bevorzugt wird Methanol, Ethanol und/oder iso-Propanol, besonders bevorzugt Ethanol und iso-Propanol, ganz besonders bevorzugt iso-Propanol, eingesetzt.

Typische Vertreter von Salzen sind die bei Behandlungsbedingungen flüssigen Salze, sogenannte "ionische Flüssigkeiten", etwa auf Imidazol-Basis.

Typische Vertreter der Gase sind beispielsweise Kohlenstoffdioxid, Dimethylether, Ethan, Propan oder Butan. Besonderer Vorteil von Gasen ist, dass sie nach der Behandlung leicht entfernt
werden können, indem der Druck im Reaktionsraum reduziert wird, wobei das Gas selbständig verdampft, so dass das Polymer in fester Form übrig bleibt.

Bevorzugt eingesetzt werden organische Lösemittel, Wasser und deren Mischungen.

Ganz besonders bevorzugt ist die Verwendung von überwiegend Wasser. "Überwiegend" meint, dass der Anteil an Wasser wenigstens 60 Prozent, bevorzugt wenigstens 70 Prozent, ganz besonders bevorzugt wenigstens 80 Prozent und insbesondere wenigstens 90 Prozent beträgt, beispielsweise 95, 99,9 oder gar bis zu 100 Prozent.

Gemäß der vorliegenden Erfindung erfolgt die Polymerisation in einer wässrigen Flüssigkeit.

Wasser kann Wasser unterschiedlicher Qualität sein: Wasser technischer Qualität, Wasser natürlich vorkommender Qualität wie Oberflächenwasser, Flusswasser und Grundwasser sowie gereinigtes Wasser. Gereinigtes ("reines") Wasser kann durch Reinigungsmethoden wie ein- oder mehrfache Destillation, Entsalzung, Diffusion, Adsorption, mittels lonentauscher sowie Aktivkohle und anderen Absorbern, mittels eines Filtrationsverfahrens wie Ultrafiltration und Dialyse gereinigt werden. Als "reines" Wasser wird dabei üblicherweise einfach oder mehrfach destilliertes Wasser sowie vollentsalztes Wasser bezeichnet.

Die erfindungsgemäße Herstellung erfolgt im Falle der löslichen Polymere bevorzugt in Lösung, bei wasserlöslichen Polymeren bevorzugt in wässriger Lösung, bevorzugt in reinem Wasser. Im Falle unlöslicher Polymere erfolgt die Herstellung bevorzugt als Fällungspolymerisation in Wasser, bevorzugt in reinem Wasser.

Diese Herstellmethoden sind dem Fachmann bekannt.

Als Initiator für die radikalische Lösungspolymerisation von Vinyllactamen wie insbesondere des N-Vinylpyrrolidons kommen die üblichen, dem Fachmann bekannten radikalbildenden Initiatoren für eine radikalische Lösungspolymerisation in Betracht.

Bevorzugte Polymerisationsinitiatoren umfassen alle dem Fachmann bekannten Initiatoren für die radikalische Polymerisation von Vinylmonomeren, insbesondere Vinyllactame, wie wasserlösliche und wasserunlösliche, bevorzugt wasserlösliche, Peroxide und Hydrogenperoxide, sowie Azo-Verbindungen, wie Wasserstoffperoxid, tert.-Butylhydroperoxid, tert.-Amylhydroperoxid, Cumolhydroperoxid, Pinanhydroperoxid, Peroxo-dischwefelsäure und ihre Salze, insbesondere ihre Alkalimetall- oder Ammoniumsalze, sowie Percarbonate und Peroxoester in Betracht. Ebenso möglich sind Redoxinitiatoren, beispielsweise Metalle mit Peroxiden, (Hydro)Peroxide mit Reduktionsmitteln wie Ascorbinsäure, Sulfite und ähnliches, sowie alle in US 5,262,171 offenbarten Initiatoren. Weitere bekannte Azo-Initiatoren sin Azobisisobutyronitril und dessen Derivate, etwa die von der Firma WAKO als "V50", "V 59" und "V 601" bekannten Substanzen. Bevorzugt eingesetzt werden Wasserstoffperoxid, tert.-Butylhydroperoxid und Di-tert-butylperoxid, insbesondere für die niedermolekulareren Polymere. Für die höhermolekulareren Polymre bevorzugt sind insberondere Azo-initiatoren, wie die genannten V59 und V601. Wasserstoffperoxid ist als Initiator besonders bevorzugt, insbesondere zur Polymerisation von N-Vinylpyrrolidon als einzigem Monomer und insbesondere für K-Werte zwischen 15 und 40.

Der Initiator wird in Mengen von 0,01 bis 10 Gewichtprozent, bevorzugt 0,05 bis 5 Gewichtsprozent, besonders bevorzugt von 0,1 bis 3 Gewichtprozent und ganz besonders bevorzugt von 0,2 bis 1 Gewichtprozent, bezogen auf die Menge an Monomeren eingesetzt.

Bei den bisher üblicherweise verwendeten Verfahren zur Polymerisation mittels Wasserstoffperoxid in wässrigen Lösungen, meist in Wasser als alleinigem Lösemittel, sind mehrere Probleme bekannt:
So bildet sich durch einen - postulierten - Zerfallsprozess aus Vinyllactam, insbesondere Vinylpyrrolidon, und Wasserstoffperoxid Ameisensäure als eines der Endprodukte, die den pH-Wert während der Polymerisation absinken lässt. Dadurch findet ab einem pH-Wert von etwa unterhalb 5,5 eine langsame, bei niedrigeren pH-Werten immer schnellere Hydrolyse des Vinyllactams, insbesondere des Vinylpyrrolidons, statt. Dabei entsteht das freie Lactam (bei Vinylpyrrolidon entsteht 2-Pyrrolidon). Tatsächlich beobachtet wird eine solche Hydrolyse mit der Bildung von signifikanten Mengen an freiem Lactam von bis zu 5 Gewichtprozent im Polymer.

Um dies zu kompensieren wurde schon in den 1940ern die Zugabe einer Base während der Polymerisation eingeführt, die die Bildung von Ameisensäure kompensieren soll. Durchgesetzt als Base hat sich Ammoniak, unter anderem wegen der geringen Kosten und wegen seiner leichten Entfernbarkeit aufgrund thermischer Flüchtigkeit. Allerdings entsteht bei der Verwendung von Ammoniak bei nicht optimaler Prozessführung Hydrazin, das toxisch ist. Solchermaßen erhaltenes Polymer ist praktisch unverkäuflich.

Dies wurde kompensiert über eine verbesserte Prozessführung und den Einsatz von Katalysatoren, die den Zerfall von Wasserstoffperoxid besser steuerbar machten. Durchgesetzt hat sich dabei die Verwendung von Kupfer-(II)-Salzen wie Kupferdichlorid und Kupfersulfat. Kupfer-(II) bildet mit Ammoniak in Wasser einen blauen Aminkomplex. Dieser bildet sich in situ während der Reaktion oder kann auch gleich als solcher eingesetzt werden.

Um die Ameisensäure-Bildung und damit die Hydrolyse der zu polymerisierenden säurelabilen Monomeren wie der Vinyllactame bei einer Batch- und Semi-Batch-Polymerisation zu unterdrücken, ist es erfindungsgemäß von Vorteil, nur einmalig zu Anfang und über einen kurzen Zeit-raum die Initiator-Menge zum Starten der Polymerisation zuzugeben:
Die Zugabe des Initiators, bevorzugt Wasserstoffperoxid, im Schritt a) kann demnach als Einzelgabe erfolgen, die schnellstmöglich zur Vorlage im Reaktionsbehälter zugegeben wird, oder als Zulauf, der über einen im Verhältnis zur gesamten Polymerisationsdauer kurzen Zeitraum von wenigen Minuten bis maximal 60 Minuten, bevorzugt maximal 45 Minuten, besonders bevorzugt maximal 30 Minuten wie beispielsweise 10, 15 oder 20 Minuten, zudosiert wird.

Nach Beendigung der Polymerisation bleibt dann eine Restmenge an N-Vinylpyrrolidon häufig von weniger als 10, meist jedoch mehr als 50 ppm und selten bis zu 2000 ppm erhalten, die wegen der toxischen Eigenschaften weiter abgesenkt werden sollte.

Die Absenkung dieser Restmenge erfolgt dann im erfindungsgemäßen Schritt e) mittels Einwirkung einer Schwefelkomponente.

Der erfindungsgemäße Schritt e) der Behandlung mit Schwefelkomponente erfolgt im Anschluss an die Polymerisation a)/b). Die Polymerisation kann, muss aber nicht eine Nachpolymerisation c) umfassen. Ist eine Nachpolymerisation vorgesehen, so erfolgt der erfindungsgemäße Schritt e) nach der Nachpolymerisation c).

Bei einer Polymerisation in einem organischen Lösungsmittel oder in einer Mischung aus organischem Lösemittel und Wasser kann es sich empfehlen, zunächst das organische Lösungsmittel zumindest teilweise oder vollständig gegen Wasser auszutauschen (erfindungsgemäßer Schritt d) und danach die Behandlung (Schritt e) durchzuführen.

Soll das organische Lösemittel zuerst ganz oder teilweise entfernt werden mittels thermischer Destillation, so findet die Behandlung im Schritt e) bevorzugt nach einer solchen thermischen Destillation statt.

Ist eine Wasserdampfdestillation vorgesehen, etwa zum teilweisen, vollständigen, nahezu voll-ständigen oder wenigstens überwiegenden Austausch des organischen Lösemittels durch Wasser, so findet die erfindungsgemäße Behandlung e) bevorzugt erst nach dieser Wasserdampf-destillation statt.

Ist sowohl eine thermische als auch eine daran anschließende Wasserdampfdestillation vorgesehen, so findet die erfindungsgemäße Behandlung vor oder nach der Wasserdampfdestillation, bevorzugt nach der thermischen Destillation und besonders bevorzugt erst nach der Wasserdampfdestillation statt.

Ist folglich eine Reinigung mittels Strippung mit Gas, thermische Destillation und/oder Wasserdampfdestillation vorgesehen (Schritt d)), so erfolgt der Schritt e) insbesondere nach diesem Schritt d).

Die mittels Schritt e) zu behandelnden Polymerlösungen oder -dispersionen weisen üblicherweise einen Feststoffgehalt von 5 bis 80 Gew.-%, bevorzugt 5 bis 60 Gew.-% auf. Bei Dispersionen beträgt der Feststoffgehalt besonders bevorzugt 5 bis 25 Gew.-% und insbesondere 8 bis 15 Gew.-%. Man kann solche Lösungen oder Dispersionen verwenden, wie sie direkt aus der Herstellung der Polymeren erhalten werden wie etwa im Lösungsmittel der Polymerisation oder der Nachpolymerisation oder deren Lösungen oder Dispersionen nach einem ganz oder teilweise Lösemittelaustausch, etwa durch thermische Destillation oder Wasserdampfdestillation. Es ist aber prinzipiell auch möglich, feste Polymere aufzulösen oder zu dispergieren und anschließend erfindungsgemäß zu behandeln.

Bevorzugt erfolgt die erfindungsgemäße Behandlung in wässrigen Lösungen oder in wässrigen Dispersionen. Besonders bevorzugt werden diese wässrigen Lösungen oder Dispersionen di-rekt aus der Polymerisation (Schritt a/b), der der Polymerisation optional nachgeschalteten Nachpolymerisation c) oder der der Polymerisation und gegebenenfalls der Nachpolymerisation nachgelagerten Reinigung im Schritt d) erhalten und eingesetzt.

Der Festgehalt der zu behandelnden Lösungen und Dispersionen kann, falls gewünscht, etwa zur besseren Durchmischung, verringert werden durch Zugabe eines geeigneten Lösemittels. Die Feststoffkonzentration kann erhöht werden durch Entfernen von Lösemittel, etwa durch thermische Destillation.

Nach dieser erfindungsgemäßen Behandlung mit Schwefelkomponente im Schritt e) kann die Polymerlösung gegebenenfalls einen erneuten Reinungsschritt d) durchlaufen, beispielsweise durch Strippen etwa mit Wasserdampf, um beispielsweise überschüssige Schwefelkomponente, insbesondere Schwefeldioxid, zu entfernen. Bevorzugt wird eine solche Wasserdampfstrippung nach dem Schritt e) durchgeführt.

Die erfindungsgemäßen bevorzugten Ausführungsformen 1 bis 84 werden daher bevorzugt so ausgeführt, dass nach der Behandlung mit Schwefelkomponente, insbesondere mit Schwefeldioxid, überschüssige Schwefelkomponente wie Schwefeldioxid durch eine Strippung, insbesondere mit Wasserdampf, aus der Reaktionsmischung entfernt wird.

"Überschüssig" bedeutet, dass Mengen an Schwefelkomponente, bevorzugt Schwefeldioxid, soweit entfernt werden, bis die als im Rahmen dieser Erfindung als bevorzugt beschriebenen, im Polymer verbleibenden Mengen erreicht werden. Die Mengen an Salzen der schwefligen Säure sind die dem Schwefeldioxid äquimolare Mengen.

Bei den erfindungsgemäßen Ausführungsformen wie insbesondere den bevorzugten Ausführungsformen 1 bis 84 beginnt die Reinigung im erneuten Schritt d) nach Schritt e) mittels Strippung, bevorzugt mit Wasserdampf, besonders bevorzugt unmittelbar nach Zugabe der Schwefelkomponente oder mit zeitlicher Verzögerung von bis zu vier Stunden, bevorzugt bis zu drei Stunden, besonders bevorzugt bis zu zwei Stunden, ganz besonders bevorzugt bis zu einer Stunde und insbesondere bis zu 30 Minuten.

Die Dauer dieser Strippung, insbesondere mit Wasserdampf, beträgt 10 bis 150 Minuten, bevorzugt 20 bis 120 Minuten, besonders bevorzugt 30 bis 90 Minuten, beispielsweise 45, 60 oder 75 Minuten.

Die Dauer der Strippung hat Einfluss auf die geruchlichen Eigenschaften der erhaltenen Polymere: Besonders guter, d.h. neutraler, Geruch wird bei einer Dauer von etwa 60 Minuten erreicht. Aber auch 30 Minuten können ausreichend sein, wenn ein leichter "Eigengeruch" der Polymere in der beabsichtigten Anwendung toleriert werden kann.

Je nach Ansatzgröße kann die tatsächlich notwendige Dauer der Strippung variieren. Die angegebenen Werte gelten für Ansätze wie in den Beispielen gezeigt bis zu mehreren 100 Kilogramm. Die jeweils notwendige Zeit kann der Fachmann leicht ermitteln anhand einzelner Versuche ausgehend von den im Rahmen dieser Erfindung gezeigten Versuche und gemachten Angaben.

Nach der Durchführung des erfindungsgemäßen Schritts e) und optionaler erneuter Reinigung d) etwa mittels Strippung nach Schritt e) kann als Schritt f) die Zugabe einer Base zur Einstellung eines gewünschten pH-Wertes, der höher ist als während des Schritts e), erfolgen.

Geeignete Basen sind alle dem Fachmann zur Einstellung von Polymerlösungen geeigneten Basen. Geeignet sind beispielsweise alle sekundären Amine (etwa die in EP 1950230 A1 im Absatz [0036] offenbarten), tertiären Amine sowie primäre, sekundäre und tertiäre Alkanolamine, Dialkanolamine und Trialkanolamine, wobei alle Amine jeweils bevorzugt nur C1 bis C6-Alkyl-Ketten, die ein oder mehrere Alkoholgruppen tragen können, aufweisen, wie Monoethanolamin, Diethanolamin, Triethanolamin oder Triethylamin. Außerdem geeignet sind beispielsweise Ammoniak und basische Ammoniumsalze, basische Carbonate wie Ammonium-, Natrium- und Kaliumcarbonat und -hydrogencarbonat, Natrium- und Kaliumhydroxid und deren insbesondere wässrige Lösungen, Guanidin und dessen Derivate und Salze wie Guanidincarbonat.

Bevorzugt finden Ammoniak, Diethanolamin, Triethanolamin, Triethylamin und Ammonium-, Natrium- und Kaliumhydroxid und -(hydrogen)carbonat Anwendung. Besonders bevorzugt ist Ammoniak, Diethanolamin, Triethanolamin, Triethylamin und Ammoniumcarbonat und -hydrogencarbonat. Ganz besonders bevorzugt ist Ammoniak, Triethanolamin und Triethylamin. Insbesondere bevorzugt ist die Verwendung von Ammoniak als Base im Schritt f), insbesondere in den Ausführungsformen 2 bis 84, in denen der Schritt f) durchgeführt wird.

Nach dem erfindungsgemäßen Schritt e) und nach der optionalen Zugabe einer Base zur Einstellung des pH-Wertes (Schritt f) kann eine Reinigung der erhaltenen Polymerlösung mittels Filterung (Schritt g) erfolgen.

Die Polymerlösung kann durch dem Fachmann bekannte Filtermedien und Filtermethoden von Inhaltsstoffen befreit oder diese Inhaltsstoffe abgereichert werden. Geeignet sind physikalische Filtermethoden und chemische Filtermethoden. Zu nennen sind insbesondere mechanische Filter wie Sieb-, Maschen- und Vliesfilter, über die Feststoffe je nach gewählter mittlerer Porengröße und Porengrößenverteilung abgetrennt werden können. Als chemische Filter sind insbesondere lonentauscher und Adsorbentien zu nennen, die beispielsweise Metallionen oder organische Inhaltsstoffe durch Komplexierung und/oder Reaktion mit dem Filtermedium abtrennen. Ebenso denkbar sind physikalische Filter wie die Adsorption von Inhaltsstoffen etwa an Aktivkohle. Weiterhin denkbar ist die Filterung mittels Osmose-, Umkehrosmose, Ultra- und Dialysemembranen. Von allen diesen Methoden bevorzugt sind die Filterung über mechanische Filter, die Filterung mittels Adsorption und die Filterung mittels Membranfiltern. Besonders bevorzugt ist die Filterung mittels Adsorption und mittels mechanischer Filter, insbesondere mittels mechanischer Filter.

Durch die Reinigung wird das erfindungsgemäß erhaltene Polymer bevorzugt so konditioniert, dass Anteile im Polymer, die wenig löslich oder geringer löslich sind als "wenig löslich" (Definition der Löslichkeit gemäß DAB 10, dem deutschen Arzneibuch Ausgabe 10, wie etwa im Roempp zitiert), aus dem Polymer abgetrennt werden, so dass deren Anteil weniger als 500 ppm, bevorzugt weniger als 150 ppm, besonders bevorzugt weniger als 100 ppm, ganz besonders bevorzugt weniger als 70 ppm und insbesondere weniger als 50 ppm je kg Polymer bezogen auf den Polymerfestgehalt beträgt. Die Mengenangaben beziehen sich dabei auf einen Filter mit der mittleren Porengröße von 5 Mikrometern, bevorzugt von 2 Mikrometern, und einer engen Porengrößenverteilung, die beispielsweise einer Gauss-Verteilung entspricht oder, bevorzugt, um 10 Prozent, bevorzugt 20 Prozent, enger als eine Gauss-Verteilung ist. Der Anteil an wenig oder unlöslichen Anteilen wird üblicherweise als "Gelgehalt" bezeichnet: Ein Polymer würde demnach beispielsweise einen "Gelgehalt von weniger als 70 ppm" aufweisen, wenn die Menge an abtrennbaren Anteilen weniger als 70 ppm beträgt (bezogen auf die Angabe der Porenweite und des Filtermediums und Filterbedingungen).

Geeignete Messmethoden sind dem Fachmann bekannt, beispielsweise aus EP 1 950 230 A1, auf dessen Offenbarung bezüglich Filtrationsrückstand und dessen Bestimmung hier vollumfänglich Bezug genommen wird.

Ist eine Trocknung h) vorgesehen, so findet der erfindungsgemäße Schritt e) vorzugsweise vor der Trocknung statt. Es ist prinzipiell aber auch möglich, erneut aufgelöstes oder erneut dispergiertes Polymerpulver dem erfindungsgemäßen Schritt e) zu unterwerfen. Wird erneut aufgelöstes/dispergiertes Polymer dem Schritt e) unterworfen, so können die weiteren Schritte d), f), g) und h) gegebenenfalls ebenfalls erneut angewendet werden.

Zur Trocknung sind prinzipiell alle dem Fachmann bekannten Methoden geeignet, wie die Trocknung durch Versprühen in Luft oder einem Gas (wie Sprühtrocknung, auch mittels Sprühscheiben und sogenannten Düsenbesen, die Sprühwirbelschichttrocknung, Sprühagglomeration und Stromtrocknung), Trocknung auf Kontaktflächen (wie Walzentrocknung, Bandtrocknung und Schaufeltrocknung) und die Trocknung mittels Vakuum wie Gefriertrocknung und Gefrier-konzentrierung oder die Trocknung mittels Strahlung wie Mikrowellen. Für die Walzentrocknung sind prinzipiell alle dem Fachmann bekannten Walzentrockner (im Englischen als "drym dryer" bekannt) verwendbar, etwa solche mit einer oder zwei Hauptwalzen und keiner bis zu sechs, bevorzugt zwei bis fünf, etwa drei oder vier Walzen, besonders bevorzugt vier oder fünf, insbesondere fünf Walzen. Diese Satellitenwalzen weisen üblicherweise einen deutlich kleineren Durchmesser als die Hauptwalze auf. Die Satelittenwalzen dienen unter anderem als Auftragswalze(n) und als Walze(n) zur Komprimierung der Polymerschicht auf der Hauptwalze. Die Anordnung der Satellitenwalzen ist prinzipiell frei wählbar. Geeignete Anordnungen sind dem Fachmann bekannt. Geeignete Anpassungen an die zu trocknenden Polymerlösungen sind dem Fachmann geläufig. Anordnungen mit üblicherweise zwei bis sechs Satellitenwalzen weisen üblicherweise obenliegende Satellitenwalzen auf, das heißt, die kleinen Walzen liegen im oberen Bereich der Hauptwalze. "Oberer Bereich" bedeutet, dass die Achsen der Satellitenwalzen oberhalb der Achse der Hauptwalze angeordnet sind. Denkbar ist allerdings auch, dass die Achse der letzten Walze insbesondere bei drei und mehr Satellitenwalzen auch zwischen der Achse der Hauptwalze und der unteren Begrenzung der Hauptwalze angeordnet sein kann. Die ersten zwei bis drei, selten vier Walzen bilden dabei ein bis drei "Sümpfe" aus, in die Polymerlösung dosiert wird. Durch die Komprimierung und/oder den mehrfachen Auftrag von Polymerlösung/-dispersion auf die gleiche Stelle der Hauptwalze bei derselben Umdrehung (d.h. mehrere Schichten Polymerlösung übereinander) können höhere Dichten des erhältlichen Polymerfeststoffes erreicht werden. Die seitliche Abdichtung der Sümpfe zwischen Satellitenwalzen und Hauptwalze ist dem Fachmann bekannt, etwa durch geeignete Bleche, Schieber usw.

Prinzipiell bekannt und im Rahmen der Erfindung ausführbar ist auch die Anordnung von zwei gegenläufigen Hauptwalzen, die zwischen den Hauptwalzen einen Sumpf bilden (können). Die Anordnung von Satellitenwalzen kann dem gleichen Muster folgen wie für die Anordnungen mit einer Hauptwalze beschrieben. Üblicherweise jedoch kommen solche Doppelwalzentrockner mit weniger Satellitenwalzen je Hauptwalze aus, üblicherweise meist nur ein bis drei, bevorzugt ein oder zwei Satellitenwalzen je Hauptwalze. Die Sümpfe werden gebildet durch zwei Satellitenwalzen die beiden Hauptwalzen und die Kombinationen daraus. Denkbar, jedoch selten für Polymerlösungen verwendet, ist die Anordnung einer oder zwei Auftragswalzen (Satellitenwalzen) im unteren Bereich einer einzelnen Hauptwalze oder die Verdoppelung dieser Anordung mit zwei Hauptwalzen.

Die Technik der Walzentrocknung als solches sowie die vielen möglichen Ausführungsformen, die verschiedenen Arten und Anzahl der Aufträge, die verschiedenen bekannten und denkbaren mechanischen, optischen, elektrischen und elektronischen Sensoren etwa zur Füllstandshöhenmessung und -steuerung des "Sumpfes"/der "Sümpfe" sind dem Fachmann hinlänglich bekannt, etwa aus einer sechzehnseitigen Broschüre "Drum Dryers" des Maschinenherstellers Royal-GMF Gouda ("Goudsche Machinefabriek B.V."), Niederlande, von 1995, insbesondere die Zeichnungen darin auf Seiten 4,5 und 14, aus "Drum Drying", J.Tang, H.Feng, G-Q-Shen in "Encyclopedia of Agricultural, Food and Biological Engineering", 2003, Marcel Dekker, im Handbook of Industrial Drying, 2007, CRC/Taylor&Francis und weiteren Standardwerken auf dem Gebiet der Chemischen Ingenieurstechnik, der Verfahrenstechnik und des Maschinenbaus.

Im Rahmen dieser Erfindung bevorzugte Verwendung finden Walzentrockner mit einer Auftragswalze und vier oder fünf, besonders bevorzugt fünf oben liegenden Satellittenwalzen und einem oder zwei, bevorzugt zwei Sümpfen, zur Trocknung von Polymerlösungen, bevorzugt wässrigen Polymerlösungen, von Vinyllactampolymeren, bevorzugt Vinylpyrrolidonpolymeren, besonders bevorzugt Polyvinylpyrrolidon, mit K-Werten von 10 bis 150, bevorzugt 50 bis 130, besonders bevorzugt wenigstens 60, ganz besonders bevorzugt wenigstens 80 wie beispielsweise 85, 90, 95, 100, 110 oder 120, und besonders bevorzugt bis zu 120 und ganz besonders bevorzugt bis zu 100.

Bevorzugt ist, insbesondere für Polymere mit Molmassen bis etwa 200.000 g/mol gewichtsmittlerer Molmasse ("niedermolekularere Polymere"), die Sprühtrocknung durch Versprühen in ein warmes Gas oder warme Luft.

Für Polymere höherer Molmassen als etwa 200.000 g/mol gewichtsmittlerer Molmasse ("höhermolekularere Polymere") ist die Trocknung auf warmen Kontaktflächen bevorzugt wie die Walzentrocknung.

Allerdings können auch Polymere mit Molmassen unter 200.000 g/mol mittels Trocknung auf warmen Kontaktflächen sowie Polymere mit Molmassen über 200.000 g/mol gewichtsmittlerer Molmasse durch Versprühen, etwa mittels Sprühtrocknung getrocknet werden.

Die Anpassung der jeweiligen Polymerlösungen oder -dispersionen etwa durch Anpassung des Feststoffgehaltes an die zu verwendende Trocknungsmethode ist dem Fachmann hinlänglich bekannt. Zu hohe Lösungviskositäten bereiten meist Probleme beim Versprühen, weshalb höhermolekulare Polymere in verdünnteren Lösungen versprüht werden müssen, während niedermolekularere Polymere auch noch bei höheren Feststoffgehalten als die höhermolekulareren Polymere versprüht werden können. Ebenso können niedermolekularere Polymere bei höheren Feststoffgehalten mittels Kontaktflächen, etwa Walzentrocknung, getrocknet werden, während dort zu geringe Feststoffgehalte Probleme verursachen können, weil die Lösungen der niedermolekulareren Polymere auf der Kontaktfläche oft zu dünnflüssig sind und keine ausreichend dicken Schichten geben. Daraus ergibt sich als mögliches Problem etwa eine zu niedrige Dichte (und damit ein zu großes Packungsvolumen je Kilogramm Polymer), eine zu niedrige Raum-Zeit-Ausbeute und zu hohe Kosten für Produktion und Verpackung.

Ein besonderer Vorteil des vorliegenden Verfahrens ist es, dass die Polymerisation bei hohen Monomerkonzentrationen durchgeführt und somit Polymerlösungen mit hohen Feststoffkonzentrationen erhalten werden können. Das Verfahren kann bevorzugt bei Monomerkonzentrationen von wenigstens 30 Gewichtprozent, besonders bevorzugt wenigstens 35 Gewichtprozent und ganz besonders bevorzugt von wenigstens 40 Gewichtprozent und insbesondere von wenigstens 45 Gewichtprozent wie bei 50 oder gar 55 Gewichtprozent durchgeführt werden bei der Herstellung von Vinyllactam-Polymeren, insbesondere PVP, mit K-Werten von 10 bis 50, bevorzugt 20 bis 40 und besonders bevorzugt 25 bis 35. Dabei treten übliche Probleme wie zu hohe Viskositäten, die das Rühren und damit die Durchmischung der Reaktionsansatzes erschweren, nicht auf oder nur in sehr geringerem Maße als bei den bisher bekannten Verfahren. Selbstverständlich kann die Polymerisation auch bei niedrigeren Feststoffgehalten unter 30 Gewichtprozent, etwa 25, 20, 15 oder nur 10 Gewichtprozent durchgeführt werden, wobei dann die wirtschaftlichen Vorteile aufgrund der höheren Konzentration entsprechend geringer ausfallen.

Je niedriger der K-Wert des herzustellenden Polymeren ist, desto höher kann die Feststoffkonzentration gewählt werden. Dieser Zusammenhang ist dem Fachmann hinlänglich bekannt. An-passungen des vorliegenden erfindungsgemäßen Verfahrens bezüglich der Festgehalte an die gewünschten K-Werte kann der Fachmann folglich leicht und ohne erfinderisches Zutun durch-führen. Bei K-Werten von 25 bis 35, insbesondere bei PVP, sind beispielsweise Feststoffgehalte von 30 bis 50, bevorzugt von 35 bis 45 Gewichtprozent mit dem vorliegenden Verfahren leicht realisierbar, ohne gewünschte Polymereigenschaften zu kompromittieren oder nachteilige Prozessparameter in Kauf nehmen zu müssen.

Ein weiterer besonderer Vorteil des vorliegenden Verfahrens ist es, dass die Polymerisation mit kurzen Reaktionszeiten auskommt, ohne dass die sonst üblichen Herangehensweisen wie sehr hohe Initiatorkonzentrationen und/oder hohe Temperaturen durchgeführt werden müssen. Durch solche Maßnahmen verursachte bekannte Probleme wie Gelbfärbung, Gelpartikelbildung und breite Molmassenverteilungen (oft bedingt durch Abweichungen von der Linearität der Polymerketten bei unvernetzten Polymeren, wobei die Abweichungen durch unerwünschte Nebenreaktionen wie Verknüpfung von Polymerketten oder durch Verunreinigung mit vernetzenden Monomeren hervorgerufen werden können), hohe Anteile an Nebenkomponenten und/oder unerwünschten Zerfallsprodukten wie Ameisensäure-, Formiat- und Lactam- wie etwa Pyrrolidon-Gehalte werden mit dem vorliegenden Verfahren vermieden. Die im Rahmen des erfindungsgemäßen Herstellverfahrens benötigten Reaktionszeiten betragen üblicherweise weniger als 6 Stunden für die Polymerisation, bevorzugt weniger als 5 Stunden, besonders bevorzugt weniger als 4 Stunden, ganz besonders bevorzugt weniger als 3 Stunden wie beispielsweise 2 Stunden und weniger bereits bei Reaktionsansätzen von mehr als einer Tonne.

Dadurch werden mittels des erfindungsgemäßen Herstellverfahrens bei hohen Raum-Zeit-Ausbeuten Polymere sehr guter Qualität bereitgestellt, die beispielsweise geringere Werte für Farbe, Geruch, Viskosität, Trübung und Gelgehalt aufweisen als bisher bekannte Polymere. Insbesondere weisen die erfindungsgemäß erhältlichen Polymerem, insbesondere Polymere erhältlich nach den bevorzugten Ausführungsformen 1 bis 84, bei Lagerung gleichbleibendere Werte für Farbe, Geruch, Viskosität, Trübung und Gelgehalt auf als die bisher bekannten Polymere. Insbesondere die Farbe und der Geruch sind besonders gering im Vergleich zu Polymeren des Stands der Technik.

Weiterhin kann das vorliegende Verfahren bei verschiedenen Drücken durchgeführt werden. Die Polymerisation kann unter Unterdruck, Normaldruck (Umgebungsdruck) wie auch unter Überdruck ausgeführt werden. Prinzipiell sind alle Druckbereiche möglich, wobei die Begrenzung im Wesentlichen durch die technische Realisierbarkeit beziehungsweise der dazu nötigen Kosten gegeben ist.

Der Überdruck ist üblicherweise begrenzt durch die Druckfestigkeit der Reaktoren zur Durchführung der Reaktion beziehungsweise die Kosten für solche hochdruckfesten Reaktoren.

Unterdruck ist üblicherweise begrenzt durch die Temperatur, bei der dann noch polymerisiert werden kann, da durch Druckerniedrigung das Lösemittel, etwa Wasser, bereits bei niedrigeren Temperaturen zu sieden beginnt und daher die Initiatoren so gewählt werden müssen, dass sie entsprechende niedrige Zerfallszeiten aufweisen, um bei den niedrigen Polymerisationstemperaturen noch hinreichend Radikale zu bilden. Initiatoren mit solchen niedrigen Zerfallszeiten sind aber sicherheitstechnisch problematisch aufgrund ihrer Instabilität beim Lagern und daher in der Regel unerwünscht.

Wie dem Fachmann bekannt hängt bei einer Polymerisation unter Druck - aufgrund sicherheits-technischer Begrenzungen bei der Auslegung von Druckreaktoren für radikalische Polymerisationen von olefinisch ungesättigten Monomeren wie Vinyllactamen, beispielswiese Vinylpyrrolidon - der Feststoffgehalt der Monomeren und damit der Feststoffgehalt der Polymeren in Lösung im Reaktor auch von der maximalen Druckstufe des Reaktorbehälters ab: Umso höhere Drücke zugelassen sind, desto höhere Festgehalte sind theoretisch möglich. Die Wahl des Feststoffgehaltes und des Druckes bedingt demnach zwangsläufig auch die Wahl eines geeigneten Reaktorbehälters.

Wie dem Fachmann bekannt hängt bei einer Polymerisation bei Umgebungsdruck (das heißt in einem "offenen System", in dem der Reaktionsraum nicht von der Atmosphäre abgeschlossen ist sondern lediglich etwa durch Gasüberlagerung oder ein Ventil, das den Druck im Reaktionsbehälter auf Umgebungsdruck regelt, von der Atmosphäre getrennt ist) der in einem Polymerisationsverfahren durchführbare Festgehalt der Monomeren im Reaktor und damit der erreichbare Polymerfestgehalt von der Kühlleistung des Kühlsystems ab, mit dem der Reaktor ausgestattet ist: Bei Polymerisation im offenen System muss das Kühlsystem die Sicherheit des Systems gewährleisten können, also beispielsweise plötzliches starkes Sieden aufgrund einer plötzlichen starken Polymerisationswärme, "abfangen" können. Die Auslegung des Kühlsystems - gemeinsam mit der Viskosität der erhältlichen Polymerlösung - begrenzt demnach die Höhe des maximal möglichen Festgehalts bei Polymerisationen in offenen Systemen.

Aus diesen, sich im Wesentlichen aus praktischen Erwägungen ergebenden Begrenzungen ergibt sich ein Druckbereich von 30.000 Pascal bis zu 5 Millionen Pascal als technisch sinnvoller Bereich für Polymerisationen.

Bevorzugt findet das vorliegende Verfahren demnach in einem Druckbereich von 30.000 Pascal bis zu 5 Millionen Pascal, besonders bevorzugt von 80.000 bis 2 Millionen Pascal, besonders bevorzugt 90.000 bis 1,5 Millionen Pascal, ganz besonders bevorzugt nicht mehr als 1 Million Pascal und insbesondere nicht mehr als 800.000 Pascal, wie beispielsweise 600.000, 400.000 oder 200.000 Pascal, statt. Am meisten bevorzugt ist die Polymerisation bei Umgebungsdruck in einem offenen System wie zuvor beschrieben.

Besonders vorteilhafte Ergebnisse bezüglich der Färbung wurden überraschenderweise erhalten wenn bei Umgebungsdruck polymerisiert wird. Die erhaltenen Polymeren weisen dabei die geringste Gelbfärbung auf, sind nahezu farblos oder farblos und sind insbesondere vollständig farblos auch bei Konzentrationen von 30 bis 45 Gewichtprozent in wässriger Lösung. Die Farbangaben bei Polymergehalten in wässriger Lösung erfolgen wie jeweils angegeben. Die Festgehalte beziehen sich auf die Werte ohne Verdünnung, das heißt wie aus Polymerisation erhalten ("tel quel"), wenn nicht eine bestimmte Feststoffkonzentration angegeben ist. Die Farbangaben bestimmt mit dem Auge eine geschulten Fachmanns sind mit der Hazen-Farbzahlmessung korreliert:
"farblos" = Hazen kleiner 20; "nahezu farblos": Hazen 20 bis 30; "minimal gelblich" = Hazen größer 30 und kleiner 40; "gelblich" = Hazen 40 und kleiner 60; "gelb" = Hazen 60 und größer.

Diese Farbangaben korrelieren die Messwerte nach Hazen mit dem Farbeindruck ausgedrückt in Worten, den das menschliche Auge bei Durchsicht einer wässrigen Lösung der Polymere mit einer Schichtdicke der Lösung von 5 Zentimetern (in einem farblosen Glasgefäß) detektiert. Zu beachten ist dabei, dass leichte Trübungen - die für das menschliche Auge nicht sichtbar sein müssen, weil etwa kolloidale Trübungen von Partikeln im Submicronbereich vorliegen (dann wäre eine "Lösung" eigentlich korrekterweise als "Suspension" zu bezeichnen) - das Messergebnis dahingehend verfälschen, dass wesentlich höhere Hazen-Werte gemessen werden, die einer massiven Gelb- oder gar Braunfärbung entsprechen, die Lösung für das menschliche Auge aber dennoch farblos oder nahezu farblos ist.

Da die Farbe für das menschliche Auge die relevante Größe bei der Anwendung solcher Polymere ist, gilt daher im Rahmen dieser Erfindung die Farbangabe in Worten als relevante Größe. Die Messwerte gelten als Unterstützung. Einzelne Abweichungen der Hazen-Farbzahl nach oben, obwohl die Farbe fürs menschliche Auge wesentlich geringer ist als es dem Hazen-Wert entsprechen würde, sind Hinweise auf für den Menschen meist nicht sichtbare Trübungen.

Ein besonderer Vorteil des vorliegenden Verfahrens ist es jedoch, dass sowohl die Farbe fürs menschliche Auge als auch die gemessenen Hazen-Werte in der Regel sehr gering ausfallen. Dies bedeutet, dass sowohl die Färbung als auch die Trübung durch kolloidal verteilte Feststoffe (wie Gelpartikel) bei den erfindungsgemäßen Ausführungsformen A1, A2 und 1 bis 84 und insbesondere bei den bevorzugten und den stärker bevorzugten Ausführungsformen besonders gering ausfallen.

Wird dagegen bei Überdruck polymerisiert, so sind mit dem vorliegenden Verfahren Festgehalte bis 60 Gewichtprozent , bevorzugt bis 55 Gewichtprozent und besonders bevorzugt bis 50 Gesichtsprozent möglich bei Drücken von bevorzugt bis zu 1,6 Millionen Pascal, besonders bevorzugt von bis zu 1 Million Pascal, und ganz besonders bevorzugt von bis zu 800.000 Pascal.

Je höher der Druck desto stärker ist jedoch auch die Färbung des Polymeren, weshalb das vorliegende Verfahren am meisten bevorzugt bei Umgebungsdruck durchgeführt wird.

Je nach Menge und Art der zugesetzten Base im Schritt b) variiert der pH-Wert, der zu Anfang und zum Ende der Polymerisation gemessen wird.

Je nach zugesetzter Menge und Art der Base und eingesetztem Initiator sinkt der pH-Wert im Laufe der Reaktion ab und kann Werte bis zu etwa 4 betragen. Diese Absenkung wird insbesondere bei der Verwendung von Wasserstoffperoxid als Initiator beobachtet. Das Ende der Polymerisationszeit ist damit - da ab pH-Werten unterhalb etwa 5,5 eine saure Hydrolyse der Vinyllactame beginnt - bereits eine beginnende Restmonomer-Hydrolyse. Soll am Ende der Polymerisationszeit der pH-Wert ebenfalls noch über 5,5 oder- bevorzugt - 6 liegen, um eine saure Hydrolyse gegen Ende auszuschließen, und damit die saure Hydrolyse - falls gewünscht - gezielt starten zu können, so ist die Menge an Base zu erhöhen. Anhand einzelner Versuche kann der Fachmann die geeigneten Basenmengen in Abhängigkeit der eingesetzten Base sehr leicht ermitteln.

Die Polymerisation im Schritt a)/b) wird erfindungsgemäß bevorzugt bei pH-Werten im Bereich von 5 bis 11, bevorzugt wenigstens 5,5, besonders bevorzugt wenigstens 6, ganz besonders bevorzugt wenigstens 6,5 wie beispielsweise 7, 7,5, 8, 8,5, 9 oder 9,5 durchgeführt, um eine Hydrolyse des N-Vinylpyrrolidons zu vermeiden. Besonders bevorzugt beträgt der pH-Wert zu Beginn der Polymerisation wenigstens 8, ganz besonders bevorzugt wenigstens 9 und insbesondere wenigstens 9,5 wie 10, 10,5 oder 11. Durch Einstellen eines hohen Ausgangs-pH-Wertes sinkt der pH-Wert im Laufe der Polymerisation - insbesondere mit Wasserstoffperoxid als Initiator - nicht so stark ab. Zusätzlich kann durch die Wahl der Menge und der Art der Base der pH-Wert-Abfall gesteuert werden. Erfindungsgemäß bevorzugt, insbesondere bei den erfindungsgemäßen Ausführungsformen 1 bis 84, erfolgt die Polymerisation bei hohen Ausgangs-pH-Werten von wenigstens 9, bevorzugt wenigstens 10 wie beispielsweise 10,5 oder 11, und solchen Basen und Basenmengen, dass der End-pH-Wert der Polymerisation nicht unter pH 5,5, bevorzugt nicht unter pH 6 fällt.

Entsprechende Einstellungen sind dem Fachmann durch einzelne Versuche ausgehend von den gezeigten Beispielen direkt zugänglich.

Vorzugsweise werden daher die Lösungen der Einzelkomponenten vor Beginn der Polymerisation gemäß Schritt b) mit einer Base (als Basen werden dieselben Substanzen ausgewählt wie für Schritt f) angegeben), bevorzugt wässriger Natronlauge, Natriumhydrogencarbonat, wässriger Ammoniaklösung oder auch Ammoniumhydrogencarbonat oder -carbonat auf diesen pH-Wert eingestellt durch einmalige Zugabe oder durch einen kurzzeitigen Zulauf wie zuvor beschrieben. Während der Polymerisation erfolgt kein Zusatz weiterer Basen. Alternativ und weniger bevorzugt wird der pH-Wert des Reaktionsmediums durch Zusatz einer solchen Base, bevorzugt Natriumcarbonat oder -hydrogencarbonat, wässrige Ammoniaklösung, Ammoniumhydrogencarbonat oder Ammoniumcarbonat, besonders bevorzugt Ammoniumhydrogencarbonat oder -carbonat, während der Polymerisation im Bereich von 5 bis 10 gehalten. Das Halten des pH-Wertes während der Polymerisation kann durch geschickten Zusatz jeweiliger Mengen - etwa durch vorheriges einfaches Ausprobieren in ein oder zwei Versuchen - an Base oder durch kontinuierliches oder wiederholtes, mehrmaliges Bestimmen des pH-Wertes während der Reaktion und daraufhin entsprechende Zugabe an Base zur erneuten Einstellung des gewünschten pH-Wertes erfolgen. Beide Ausführungsformen liefern ein im Rahmen der Messabweichungen gleiches Ergebnis.

Vorteilhaft an der einmaligen Zugabe von Base sind die vereinfachte Steuerung der Reaktion sowie der Verzicht auf die kontinuierliche pH-Bestimmung und damit der Verzicht auch auf die gesteuerte Zugabe von Base. Damit wird die Überwachung von zwei Prozessparametern überflüssig, was einer Kosteneinsparung entspricht und Fehlerquellen minimiert.

In einer bevorzugten Ausführungsform erfolgt die Zugabe der Base, besonders bevorzugt Ammoniumhydrogencarbonat, zu Beginn der Polymerisation im Schritt a)/b). Dies bedeutet, dass die Base bereits zu Beginn der Polymerisationsreaktion zugegeben wird, bevorzugt als Einzelgabe, die schnellstmöglich zur Vorlage im Reaktionsbehälter zugegeben wird oder als Zulauf, der über einen im Verhältnis zur gesamten Polymerisationsdauer kurzen Zeitraum von wenigen Minuten bis maximal 60 Minuten, bevorzugt maximal 45 Minuten, besonders bevorzugt maximal 30 Minuten wie beispielsweise 10, 15 oder 20 Minuten, zudosiert wird. Während der Dauer der Polymerisation erfolgt bevorzugt keine weitere Zugabe von Base. Besonders bevorzugt im Rahmen der vorliegenden Erfindung - insbesondere bei den bevorzugten Ausführungsformen 1 bis 84 - ist die Durchführung so, dass im Schritt b) der pH-Wert zu Beginn durch Zusatz von Base eingestellt wird und keine weitere Dosierung von Base während der Polymerisation erfolgt. Insbesondere bevorzugt ist dabei jeweils die Verwendung von Ammoniumhydrogencarbonat als Base im Schritt b).

Die üblicherweise gewünschten niedrigen Restmonomer-Gehalte von weniger als 100 ppm können gemäß Veröffentlichungen des Stands der Technik und gemäß allgemeinem Fachwissen durch eine optionale Nachpolymerisation erreicht werden, bei der weiterer, bevorzugt der gleiche wie auch zur Polymerisation eingesetzte, Initiator erneut zugesetzt wird: Der Zusatz weiteren Initiators kann wiederum als einmalige Gabe, als Dosierung oder als mehrmalige Gabe oder mehrmalige Dosierung oder Kombinationen davon durchgeführt werden. Üblicherweise reichen die Dosierung/Zugabe von zwei, bevorzugt nur einer weiteren Portion(en) Initiator aus, um die Restmonomere, insbesondere bei Vinyllactamen wie Vinylpyrrolidon, auf Werte von maximal 200 ppm, bevorzugt maximal 100 ppm, besonders bevorzugt maximal 50 ppm wie etwa weniger als 10 ppm bezogen auf den Polymerfestgehalt abzusenken. Die zur Erreichung solch niedriger Restmonomere notwendige Zeitdauer für die Nachpolymerisation wird allerdings in der Regel nicht abgewartet, da die Reaktorbelegungszeit zu teuer ist. Daher wird im Stand der Technik in der Regel eine solche Nachpolymerisation kürzer durchgeführt als eigentlich notwendig. Die dann noch nötige Absenkung der Restmonomere wird gemäß der Veröffentlichungen des Stands der Technik durch andere Prozesschritte, etwa die saure Hydrolyse der Monomere, durchgeführt.

Überraschenderweise ergeben sich bei Durchführung des vorliegenden Verfahrens ohne weitere Initiatorzugabe und damit ohne eine Nachpolymerisation (Schritt c) Restgehalte an Monomeren, insbesondere bei Vinyllactamen wie Vinylpyrrolidon, die so niedrig liegen, dass auf eine Nachpolymerisation verzichtet werden kann, da die Werte bereits ohne eine solche Nachpolymerisation (Schritt c)) so niedrig liegen, dass die restliche Absenkung durch den erfindungsgemäßen Schritt e) der Zugabe von Schwefelkomponente auf solche Werte von maximal 100 ppm, bevorzugt maximal 50 ppm, besonders bevorzugt maximal 10 ppm, ganz besonders bevorzugt maximal 5 ppm und insbesondere maximal 1 ppm bezogen auf den Polymerfestgehalt abgesenkt wird.

Dieser Verzicht der weiteren Zugabe von Initiator und damit der Verzicht auf Schritt c) ist insbesondere deshalb vorteilhaft, da sich herausstellte, dass die Zugabe von weiterem Initiator auch zu einer Farbvertiefung oder gar erst zu einer Färbung der Polymeren führt. Außerdem kann die benötige Prozesszeit und damit die Dauer der Reaktorbelegung verringert werden, was eine weitere Kostensenkung bedeutet.

Besonders bevorzugt ist im Rahmen dieser Erfindung daher die Durchführung mit nur einer Initiator-Zugabe zu Beginn der Polymerisationsreaktion im Schritt a) und der Verzicht auf Schritt c): Von den erfindungsgemäßen Ausführungsformen 1 bis 84 werden daher die Ausführungsformen bevorzugt, die ohne die nachträgliche Zugabe weiteren Initiators und damit ohne Schritt c) durchgeführt werden.

Es war daher naheliegend, den verbleibenden, zwar niedrigen aber dennoch unerwünschten Rest-Vinylpyrrolidon-Gehalt statt durch Initiator-Zugabe durch eine Säurebehandlung gemäß der Veröffentlichungen des Stands der Technik zu zerstören. Üblicherweise wird die Polymerlösung mit einer anorganischen oder organischen Säure auf einen pH-Wert von kleiner als 4 eingestellt. Dadurch wird das Vinylpyrrolidon im wässerigen Medium in Acetaldehyd und Pyrrolidon hydrolysiert wie es etwa im Kirk-Othmer (Abschnitt "N-Vinylamide polymers" in "Encyclopedia of Polymer Science and Technology", 2005, John Wiley & Sons, Inc) beschrieben ist.

Durch diese Säurebehandlung wird keine weitere Ameisensäure mehr nachgebildet. Das entstehende Acetaldehyd kann nachträglich durch einen Gasstrom z.B. Stickstoff, partielles Abdestillieren oder durch Wasserdampfstrippung entfernt werden. Dieses Verfahren hat aber den Nachteil, dass bei Verwendung von diversen, im Stand der Technik üblicherweise verwendeten anorganischen und organischen Säuren wie Schwefelsäure, Phosphorsäure, Salzsäure, Ameisensäure, Milchsäure eine unerwünschte tiefgelbe Verfärbung der Lösung auftritt.

Durch den erfindungsgemäßen Einsatz von Schwefelkomponente im Schritt e), bevorzugt Schwefeldioxid als wässrige Lösung, wird diese Verfärbung während der Hydrolyse nicht mehr beobachtet: Es wird eine nahezu farblose oder farblose Polymerlösung oder -dispersion erhalten. Die erhaltene Polymerlösung oder -dispersion bleibt dabei über mehrere Monate auch bei einer Lagertemperatur von Raumtemperatur (25° C) oder 40° C oder gar höher sowie auch bei höheren Feuchtegehalten oder Kombinationen von diesen Temperaturen und diesen Feuchtigkeitswerten farblich unverändert oder nahezu unverändert. Auch eine etwas höhere Stabilität in Gegenwart von Luftsauerstoff konnte beobachtet werden, weshalb durch Sauerstoff verursachte Verfärbungen und sonstige Verschlechterungen etwa bei der Viskosität von Polymerlösungen, deren Geruch und Klarheit deutlich geringer ausfallen. Ebenso werden bessere Anwendungseigenschaften und eine höhere Stabilität dieser Eigenschaften erreicht, etwa bei Haargelen und haarkosmetischen Formulierungen.

Schweflige Säure (Schwefeldioxid als wässriger Lösung) wurde bisher im Stand der Technik nie als Säure zur sauren Hydrolyse in Betracht gezogen. Einzige bekannte Verwendung ist der Zusatz von schwefliger Säure zur Stabilisierung. Diese Zugabe erfolgt gemäß DE 10 2005 00 5974 wie eingangs zitiert aber bei neutralen pH-Werten, wenigstens jedoch bei pH-Werten oberhalb von 6 und damit außerhalb der pH-Wert-Bereiche statt, bei denen eine Hydrolyse von Vinyllactam-Monomeren, insbesondere von Vinylpyrrolidon, beobachtet werden kann. Der Zusatz von Schwefeldioxid oder Sulfiten gemäß GB 836,831 zur Verringerung/Verhinderung der Gelbfärbung beim Erhitzen von Polymerlösungen ist ebenfalls keine Vorbeschreibung des erfindungsgemäßen Schrittes e), da dort die Schwefelverbindungen zu fertigen Polymeren zugesetzt werden in einem festen Mengenverhältnis zum Polymer. Dadurch wird bei den dortigen Versuchen keine Absenkung des pH-Wertes unter 5,5 und damit keine saure Hydrolyse erreicht. Die eingesetzten Mengen bewirken dort vielmehr, dass eine wässrige PVP-Lösung mit einem pH-Wert von 8 nur gering auf einen pH-Wert von 7,7 absinken. Der saure pH-Wert wird nicht alleine durch den Zusatz einer Schwefelverbindung in den dort offenbarten Mengen erreicht: Die Schwefelverbindung dient daher in GB 836,831 - wie dort als Kern der dortigen Erfindung offenbart - nur als Stabilisator beim Erhitzen, nicht aber jedoch als Säure zur sauren Hydrolyse.

Die Verwendung von Schwefeldioxid in wässriger Lösung als Säure zur Hydrolyse war demnach unbekannt. Die Entdeckung der unerwarteten Eigenschaften resultierend aus der bisher unbekannten Verwendung von Schwefeldioxid als Säure zu einer solchen sauren Hydrolyse ist das Verdienst der vorliegenden Erfindung.

Das Verfahren der vorliegenden Erfindung und insbesondere die bevorzugten Ausführungsformen 1 bis 84 werden daher so durchgeführt, dass nach einer Nachpolymerisationszeit (ohne Initiatorzugabe) von 10 Minuten bis 4 Stunden, bevorzugt 30 bis 60 Minuten, und der optionalen, bevorzugt jedoch ausgeführten Reinigung im Schritt d) mittels bevorzugt Wasserdampfstrippung die Polymerlösung erfindungsgemäß im Schritt e) mit Schwefelkomponente in wässriger Lösung, bevorzugt Schwefeldioxid in Wasser, auf einen pH-Wert von kleiner als 4 eingestellt und zwischen 40 und 150 ° C, bevorzugt 50 und 90° C gerührt wird. Dadurch werden die Restmonomere so weit abgesenkt, dass diese bei maximal 100 ppm, bevorzugt maximal 50 ppm, besonders bevorzugt maximal 10 ppm, ganz besonders bevorzugt maximal 5 ppm und insbesondere maximal 1 ppm bezogen auf den Polymerfestgehalt liegen. Dier erhaltenen Polymere weisen eine außergewöhnlich geringe Farbe auf und sind nahezu farblos oder farblos und weisen in der Regel weitere vorteilhafte Eigenschaften auf wie im Rahmen dieser Erfindung beschrieben.

Dies wird erfindungsgemäß durch Behandlung mit Schwefelkomponente erreicht, ohne jedoch das Polymer mit anderen Substanzen zu verunreinigen wie Enzyme, Schwermetalle, Metalle oder Antioxidantien, wie dies im Stand der Technik vorgeschlagen wird. Einziger zusätzlicher Inhaltsstoff ist Schwefeldioxid, dessen Salz Sulfit oder dessen oxidativen Folgeprodukte, Sulfat und Hydrogensulfat. Durch die bevorzugte Verwendung von Schwefeldioxid anstelle von Sulfi-ten werden auch keinerlei Metallionen wie Natrium, Kalium, Magnesium oder Calcium einge-bracht. Dadurch erhöht sich auch nicht der Aschegehalt der Polymere oder zumindest nicht so stark, als wenn Metallsalze eingebracht würden.

Der Aschegehalt bestimmt sich nach der dem Fachmann bekannten Bestimmung des Aschegehalts als "Sulfatasche-Gehalt". Der Aschegehalt beträgt bevorzugt höchstens 0,05 Gewichts-prozent, besonders bevorzugt höchstens 0,02 Gewichtprozent, besonders bevorzugt höchstens 0,01 Gewichtprozent und ganz besonders bevorzugt höchstens 0,005 Gewichtprozent wie beispielsweise nur 0,002 Gewichtprozent, 0,001 Gewichtprozent oder weniger. Solche niedrigen Aschegehalte von höchstens 0,02 Gewichtprozent und darunter werden insbesondere dann erhalten, wenn als Base im Schritt b) und/oder im Schritt f) eine Substanz/Substanzen eingesetzt wird/werden, die nicht zum Aschegehalt beitragen und/oder sich leicht restlos oder nahezu restlos entfernen lässt/lassen durch einfache Prozessoperationen wie Destillieren, Strippen oder Entgasen bei höherer Temperatur oder unter Anlegen eines Vakuums. Die Angabe des Aschegehalts bezieht sich dabei auf die Gesamtmasse des erfindungsgemäß erhältlichen Polymers, bezogen auf den Feststoffgehalt des Polymeren. Besonders bevorzugt als Base im Schritt b) und Schritt f) sind daher Ammoniak, Ammoniumhydrogencarbonat und Ammoniumcarbonat. Ganz besonders bevorzugt für Schritt b) ist Ammoniumhydrogencarbonat und Ammoniumcarbonat, insbesondere Ammoniumhydrogencarbonat. Ganz besonders bevorzugt für Schritt f) ist Ammoniak.

Besonderer Vorteil des vorliegenden Verfahrens und der hier offenbarten Ausführungsformen 1 bis 84 und der daraus erhältlichen Polymere ist der höhere pH-Wert der Polymerlösungen, weshalb zur Einstellung von höheren pH-Werten wie etwa zur Neutralisation geringere Mengen an Basen notwendig sind. Dies ist insbesondere deshalb vorteilhaft, da Formulierungen, wie etwa haarkosmetische Formulierungen, beispielsweise Haargele, mit weniger Base versetzt werden müssen. Dadurch wird die Salzfracht solcher Formulierungen geringer. Dies spart einerseits Kosten und eröffnet größere Flexibilität der Formulierungen durch weniger Inhaltsstoffe und -mengen.

Außerdem wurde im Rahmen der vorliegenden Erfindung beobachtet, dass Haargele, insbesondere klare Haargele, die Verdicker vom Carbomer-Typ enthalten, enthaltend die erfindungsgemäß erhältlichen Polymere, eine höhere Gelstabilität aufweisen.

In der Polymerlösung verbleiben auch nach einer Strippung üblicherweise noch Restbestände an Schwefelkomponente, die durch eine weitere, optionale Nachbehandlung mit Peroxiden wie die Zugabe von Wasserstoffperoxid weiter reduziert werden können.

Für die Farbstabilität ist es aber insbesondere von Vorteil und daher eine bevorzugte Ausführungsform insbesondere auch der Ausführungsformen 1 bis 84, wenn im Polymerisat noch gewisse Mengen an Schwefeldioxid beziehungsweise Sulfit verbleiben, die wenigstens 10 ppm, bevorzugt wenigstens 100 ppm, besonders bevorzugt wenigstens 200 ppm und ganz besonders bevorzugt wenigstens 500 ppm wie insbesondere 1000 ppm bezogen auf den Polymerfestgehalt betragen wie 300, 400, 600, 700, 800, 900, 1100, 1200, 1300, 14003 1500 oder gar 1750 ppm.

Weiterhin ist es von Vorteil, wenn die Mengen an Schwefeldioxid beziehungsweise Sulfit einen Wert von 5000 ppm, bevorzugt von 3000, besonders bevorzugt von 2000 nicht überschreiten.

Die Mengenangaben beziehen sich dabei immer auf "Schwefeldioxid", bei Sulfiten auf Schwefeldioxid-Äquivalente (d.h. berechnet als "Schwefeldioxid").

Die erfindungsgemäß erhältlichen Polymere zeichnen sich wie folgt aus:
Die Polymere sind farblos, nahezu farblos oder minimal gelblich. Die ist insbesondere der Fall auch bei PVP-Homopolymeren mit K-Werten von 20 bis 40. Diese geringe Färbung ("nahezu farblos") oder Farblosigkeit ("farblos") ist insbesondere vorhanden auch bei den bei diesen K-Werten kommerziell üblichen Polymerfeststoffkonzentrationen von 20 bis 60 Gewichtprozent in Wasser.

Die Farbe wird dabei als HAZEN-Farbzahl angegeben (auch als Platin-Kobalt-Farbzahl bezeichnet und in der DIN ISO 6271-1 beschrieben). Die Angaben zu der Farbe mittels Bestimmung durch ein geschultes Auge und dessen Korrelation zu Hazen-Farbwerten wurde bereits oben wiedergegeben.

Wichtig ist die Farbe bestimmt mit dem geschulten Auge. Der Hazen-Farbzahlwert ist lediglich eine ergänzende Angabe. Ist auch dieser Hazen-Farbzahlwert niedrig, ist folglich auch die Lösung klar ohne oder - bei minimal erhöhten Hazen-Farbzahlwerten aber mit dem Auge bestimmter Farblosigkeit - nahezu ohne Trübung.

Die erfindungsgemäß erhältlichen Polymere sind demnach minimal gelblich, nahezu farblos oder farblos, bevorzugt nahezu farblos oder farblos und besonders bevorzugt farblos, jeweils bestimmt mit dem geschulten Auge eines Fachmanns, bestimmt ans wässrigen Polymerlösungen bei einer Schichtdicke von 5 Zentimeter, bevorzugt bei hohen Polymerfeststoffkonzentrationen, wie die Polymere aus der Polymerisation und Aufarbeitung erhalten werden, insbesondere also bei den bevorzugten hohen Feststoffgehalten wie zuvor spezifiziert, und insbesondere bei den bevorzugten Ausführungsformen 1 bis 84.

Die Hazen-Farbzahlwerte weisen dementsprechend die direkt diesen Farbangaben korrelierten Werte auf wie zuvor spezifiziert.

Die erfindungsgemäß erhältlichen, insbesondere die mittels Ausführungsformen 1 bis 84 erhältlichen Polymere weisen darüber hinaus eine Stabilität der Farbe auch beim Lagern auf unter den Lagerbedingungen und Verpackungen zur Lagerung und Transport wie im Rahmen dieser Erfindung spezifiziert. Diese Stabilität ist dadurch gekennzeichnet, dass die Farbangaben sich auch beim Lagern, bei Raumtemperatur als auch bei erhöhten Temperaturen von 40 ° C oder gar 50 ° C in den genannten Verpackungen keine oder nahezu keine Verschlechterung der Farbe zeigen auch beim Lagern über einen Zeitraum von 4 Wochen, bevorzugt 8 Wochen, besonders bevorzugt 3 Monate, ganz besonders bevorzugt 5 Monate und insbesondere 1 Jahr oder gar 2 bis 3 Jahre.

"Nahezu keine" Verschlechterung bedeutet, dass sich der Wert um weniger als 20 Prozent, bevorzugt weniger als 10 Prozent und besonders bevorzugt weniger als 5% vom Ausgangswert verändert. Insbesondere tritt keine für die jeweilige Anwendung relevante Farbverschlechterung auf.

Die Polymere ergeben in Lösung, insbesondere in wässriger Lösung, eine klare Lösung ohne Trübung. Die messbare Trübung ist dabei sehr gering, bevorzugt unterhalb 3, bevorzugt unterhalb 2,5, besonders bevorzugt unterhalb 2, ganz besonders bevorzugt unterhalb 1,5 und insbesondere unterhalb 1, wie 0,9, 0,8, 0,70,6, 0,5 oder gar 0,4, 0,3, 0,2, 0,1 und geringer, bestimmt als FTU-Wert nach DIN ISO 15715 an einer 10 gewichtprozentigen, wässrigen Polymerlösung.

Die erfindungsgemäß erhältlichen, insbesondere die mittels Ausführungsformen 1 bis 84 erhältlichen Polymere weisen darüber hinaus eine Stabilität der Trübung auch beim Lagern auf unter den Lagerbedingungen und Verpackungen zur Lagerung und Transport wie im Rahmen dieser Erfindung spezifiziert. Diese Stabilität ist dadurch gekennzeichnet, dass die Trübung sich auch beim Lagern, bei Raumtemperatur als auch bei erhöhten Temperaturen von 40 ° C oder gar 50 ° C in den genannten Verpackungen keine oder nahezu keine Verschlechterung der Trübung zeigen auch beim Lagern über einen Zeitraum von 4 Wochen, bevorzugt 8 Wochen, besonders bevorzugt 3 Monate, ganz besonders bevorzugt 5 Monate und insbesondere 1 Jahr oder gar 2 bis 3 Jahre.

Die Polymere zeigen nur eine geringe oder keine anwendungstechnisch relevante, bevorzugt auch keine messtechnisch relevante, Veränderung der Viskosität einer wässrigen Lösung beim Lagern bei Raumtemperatur (25 ° C) oder, bevorzugt, auch nicht beim Lagern bei erhöhter Temperatur von bis zu 40 ° C, bevorzugt bis zu 50 ° C.

Nach dem vorliegenden Verfahren erhältliche Polymere zeigen Viskositäten ihrer wässrigen Lösungen, die im Vergleich zu Polymeren erhalten mit Verfahren nach dem Stand der Technik niedriger sind. "Niedriger" bedeutet dabei, dass die Viskositäten bis zu 5 Prozent, bevorzugt mehr als 5 Prozent, besonders bevorzugt mehr als 7 Prozent und besonders bevorzugt mehr als 10 Prozent niedrigere Viskositäten zeigen als Polymere nach dem Stand der Technik bei ansonsten identischen Messbedingungen.

Nach dem vorliegenden Verfahren erhältliche Polymere zeigen eine Stabilität der Viskosität beim Lagern, wobei die Viskosität der wässrigen Lösung nach einer Lagerzeit von wenigstens 3 Monaten, bevorzugt wenigstens 6 Monaten, besonders bevorzugt wenigstens 9 Monaten, ganz besonders bevorzugt wenigstens 12 Monaten und insbesondere von wenigstens 2 Jahren eine Veränderung der Viskosität von nicht mehr als 15 %, bevorzugt nicht mehr als 10 Prozent, besonders bevorzugt nicht mehr als 5 %, ganz besonders bevorzugt nicht mehr als 3 Prozent und insbesondere nicht mehr als 2 Prozent vom Ausgangswert aufweist wie beispielsweise nicht mehr als 1 Prozent und ändert sich beispielsweise überhaupt nicht im Rahmen der Messwertgenauigkeit. Die Messmethode dazu ist nicht wesentlich, wenn stets die identischen Messbedingungen für Vergleiche verwendet werden.

Die Viskosität einer wässrigen Lösung wird bestimmt etwa mittels Brookfield-Viskosimetern. Die Festgehalte und die zu verwendenden Spindeln variieren je nach Molmasse der zu untersuchenden Polymere. Die Messung und die geeigneten Spindeln und Gewichtskonzentrationen der Polymere je nach gewichtsmittlerem Molgewicht ist dem Fachmann als solches bekannt und in zahllosen Veröffentlichungen und Produktbroschüren, etwa denen der BASF zu Polyvinylpyrrolidonen für technische Anwendungen (Markenname "Luvitec®"), veröffentlicht und referenziert. Wichtig zur Beurteilung der Stabilität der Viskosität ist jedoch nur, dass für vergleichende Messungen stets dieselben Messbedingungen Verwendung finden. Dann ist die Art der Messung nicht wesentlich für den Vergleich zweier Messergebnisse an zwei verschiedenen Polymeren oder verschieden gealterten/gelagerten Polymerproben.

Die Lagerung findet jeweils bevorzugt in geschlossenen Gebinden statt. Besonders bevorzugt sind solche geschlossenen Gebinden gasdicht oder nahezu gasdicht und weiterhin auch bevorzugt dicht oder nahezu dicht gegenüber Wasserdurchgang.

Die Gasdurchlässigkeit wird dabei bezogen auf ein gewähltes Referenzgas, wie Sauerstoff, angegeben.

Nach Bestimmungsmethoden ASTM D3985, DIN 53380/3 weisen die Verpackungen für die Lagerung der Polymere eine Sauerstoff-Permeabilität von weniger als 0,5, bevorzugt von weniger als 0,45 und besonders bevorzugt von 0,4 cm³/(m² x d x bar) und geringer bei 23° C und 50% relativer Luftfeuchtigkeit auf.

Die Norm ASTM D3985, die zur Ermittlung der Barriereeigenschaften von Mehrschichtstrukturen verwendet wird, beschreibt die konstante Sauerstoffdurchlässigkeits-rate von EVOH und wird mittels Coulometers an Folien (Prüfkörpern) ermittelt.

Die Wasserdampfpermeabilität der Verpackungen für die Lagerung der Polymere ist nach ASTM F1249 geringer als 0,5, bevorzugt geringer als 0,4 und insbesondere bevorzugt 0,3 g/cm²xd und geringer bei einer Temperatur von 23° C und 85 % relativer Luftfeuchtigkeit.

Nach alternativer Bestimmungsmethode ist die Wasserdampfpermeation nach DIN EN 12086-Klima B kleiner als 0,5, bevorzugt kleiner als 0,4 und insbesondere bevorzugt kleiner gleich 0.35g/m²xd.

Diese Werte für Gasdurchlässigkeit und Wasserdampfpermeabilität gelten für Folienbeutel, Foliensäcke oder Gebinde, mit denen die Polymerlösung, Polymerdispersion oder der Polymerfeststoff in direkten Kontakt kommen. Solche geeigneten Verpackungsmaterialien sind aus der WO 2010/052088 A1 bekannt.

Erfindungsgemäße Polymere erhältlich nach dem vorliegenden erfindungsgemäßen Verfahren sind daher bevorzugt verpackt in Verpackungsformen, die aus Mehrschichtfolien hergestellt wurden, unter Verwendung eines Verfahrens zur Verpackung, wobei die Mehrschichtfolien, die Verpackungsformen und die Verpackungsverfahren denen der WO 2010/052088 A1 entsprechen.

Nach dem vorliegenden Verfahren erhältliche Polymere zeigen einen geringen bis nahezu nicht vorhandenen, als "typischen Eigengeruch" bezeichneten Geruch. Ist der Eigengeruch minimal, so wird der Geruch als "neutral" bezeichnet. "Muffige" Gerüche, die bei PVP und Vinyllactam-Polymeren bekannt und sehr verbreitet sind, treten bei den vorliegenden Polymeren nicht auf. Vielmehr verändert sich der geringe bis sehr geringe Eigengeruch/neutrale Geruch beim Lagern, insbesondere auch bei erhöhten Temperaturen von 40° C oder gar 50° C über Zeiträume von wenigstens 3, 6, 9, 12 oder mehr Monaten oder gar 2 Jahren praktisch nicht. Insbesondere tritt keine Geruchsverschlechterung auf. Die Bewertung des Geruchs und das Verständnis von Begriffen wie "neutraler Geruch", "Eigengeruch", "typischem Eigengeruch" und "muffigen Gerüchen" sind dem Fachmann bekannt.

Bevorzugt zeigen die nach dem vorliegenden Verfahren erhältlichen Polymere wenigstens in zwei, besonders bevorzugt in drei, ganz besonders bevorzugt in vier und insbesondere in allen fünf Eigenschaften ausgewählt aus Farbe, Geruch, Gelgehalt, Viskosität und Trübung die jeweils zuvor angegebenen erfindungsgemäßen Eigenschaften und - bevorzugt - auch die Kombinationen der jeweiligen bevorzugten Werte für diese Eigenschaften.

Bevorzugt kommt das erfindungsgemäß erhältliche Polymer, bevorzugt Polyvinylpyrrolidon mit K-Werten zwischen 20 und 35, bevorzugt in wässriger Lösung mit Polymerfeststoffkonzentrationen zwischen 15 und 60 Gewichtprozent, bevorzugt hergestellt in wässriger Lösung, bevorzugt mittels Wasserstoffperoxid als Initiator, weiterhin bevorzugt mit Ammonium(hydrogen)carbonat, besonders bevorzugt Ammoniumhydrogencarbonat, als Base vor/während der Polymerisation, und weiterhin bevorzugt mit Ammoniak als Base zur pH-Wert-Anhebung nach der Polymerisation, in kosmetischen und pharmazeutischen Zubereitungen zur Anwendung, etwa als lagerstabiles Desinfektionsmittel im Komplex mit Jod, als Bindemittel, als Texturbildner sowie die sonstigen pharmazeutisch üblichen und bekannten An-wendungsgebiete sowie als Inhaltsstoff in haarkosmetischen Zubereitungen, wie als Festiger in Haargelen, insbesondere solche mit Carbomer-Typen als Verdicker.

Weiterhin bevorzugt ist die Verwendung des erfindungsgemäß erhältlichen Polymers zur Herstellung von Membranen, insbesondere zur Trennung und Reinigung von Stoffen, insbesondere von Flüssigkeiten, wie etwa Dialysemembranen und Membranen zur Reinigung von Blut und Wasser. Typische Dialysemembranen enthalten Polysulfone und/oder Polyethersulfone und PVP, das unter anderem zur Porenbildung und Steuerung der Porengröße Verwendung findet.

Weiter sind die Polymere, wie insbesondere die Polyvinylpyrrolidone, als Waschmitteladditive (z. B. Farbübertragungsinhibitoren, Vergrauungsinhibitoren) sowie für zahlreiche technische Anwendungen (Photoresists, Verdicker, Klebstoffe, Hilfsmittel für die Textileinfärbung, Klebestifte, Metallquenchbäder, Trennung von Edelmetallen, Aufhellmittel, Komplexe mit Antioxidantien, Betonzusatzmittel, Coating von Polyolefinen/Fasern, Druckfarben, Diazotypien, elektrisch leitende Schichten, Elektrodengele, Hautklebegele, Ultraschallgele, Entfernung polyvalenter Kationen, Entfernung von Poylphenolen, Enzym- und Proteinkomplexe, Farbmischungsinhibitoren, Festbatterien, Festelektrolyte, Fischfuttergranulat, Fixateur für Parfümöle, Flexodruckplatten, Flockungsmittel, Fotographische Platten, Gasanalytik, Gipsbinden, Schmiermittel, Haftvermittler für Farbstoffe, Hydrophilisierung von Oberflächen, Ionenaustauscher, Isomerisierungsinhibitor, Schutzkolloid, Tinten, Jetinks, Kugelschreiberpasten, Katalysatoren, Kathedercoating, Keramikbinder, Kesselsteinentferner, Klebstoff für Nährböden, Komplexbildung mit organischen oder anorganischen Verbindungen zur Erhöhung der Adsorbierbarkeit/Hydrophobie, Komplexe mit Halogen, Komplexe mit Polymeren, Konservierungsmittel, Kontaktlinsen, Korrosionsschutz, Kunststoffadditive, Lackhilfsstoffe, lichtempfindliche Materialien, Lithographie, Solubilisierung, Luftfilter, Metallguss, Metallhärtung, Stabilisierung von Metallkolloiden, Metallkomplexe für reversible Saürstoffabsorption, Mikroverkapselung, Öl- und Farbstoffentfernung aus Wasser, Ölrückgewinnung, Papierhilfsmittel, Papierstreichfarben, Phasen-Transfer-Katalysatoren, Photoimaging, Pigmentdispersionen, Protonenleiter, Reinigungsmittel von Ab-wässern, Saatgutbeize, Saatgutcoating, Schmiermitteladditive, Silberhalogenid-Emulsionen, Soil Release, Stabilisierung von Peroxiden, Synthetische Fasern, Tertiäre Erdölgewinnung, Textilhilfsmittel Trennung von Kohlenwasserstoffgemischen, Viskositätsmodifizierung, wärmebeständige Schichten, wärmeempfindliche Schichten, wärmeempfindliche Widerstände, wasserlösliche Filme, Zigarettenfilter) brauchbar und zeigen in vielen Fällen gegenüber den bisher erhältlichen Polymeren positive Eigenschaften, sei es in der Anwendung, der Verarbeitung und/oder der jeweiligen Haltbarkeit der Zubereitungen, Filme und hergestellten Erzeugnisse, der Vereinfachung der Formulierung oder der Kompatibilität in Formulierungen und Anwendungen.

Die erfindungsgemäß erhältlichen, insbesondere die mittels der bevorzugten Ausführungsformen 1 bis 84 erhältlichen Polymere enthalten wie gewünscht nur sehr niedrige Ameisensäuregehalte, enzymatisch bestimmt wie in einschlägigen Veröffentlichungen, etwa Arzneibüchern, angegeben, von weniger als 2000 ppm, bevorzugt weniger als 1500 ppm, besonders bevorzugt weniger als 1200 ppm und ganz besonders bevorzugt von weniger als 1100 ppm und insbesondere weniger als 1000 ppm wie 900 ppm, 800 ppm, 700 ppm oder noch geringer, jeweils bezogen auf 100 Gewichtsprozent Polymer.

Polymer erhältlich nach dem Stand der Technik, etwa gemäß der Beispiele 1 bis 3 aus DE 11 2005 00 2719, sowie Polymere, die aus Varianten des vorliegenden Verfahrens, das aber mittels Säuren aus dem Stand der Technik anstelle von Schwefelkomponente im Schritt e) durchgeführt wurde, erhalten wurden, zeigen dagegen sehr viel höhere Werte für die erfindungsgemäß relevanten Parameter: Ameisensäuregehalte, Viskosität, Farbe, Trübung, pH-Werte, Geruch zeigen deutlich abweichende, nachteilige Werte, wie in den Beispielen gezeigt und bestimmt.

Besondere Vorteile der erfindungsgemäß erhältlichen Polymere, insbesondere bei PVP, das gemäß einer der bevorzugten Ausführungsformen erhalten wurde, sind insbesondere hohe Stabilität der Farbe auch in Haargelen und insbesondere in klaren, farblosen Gelen, die üblicherweise Verdicker des Carbomer-Typs enthalten. An diesen Gelen konnte praktisch keinerlei Verfärbung festgestellt werden auch beim Lagern bei Raumtemperatur oder höheren Temperaturen.

Die Gelstabilität solcher Haargele ist auch bei Lagerung bei erhöhter Temperatur besser als bei bisherigen Polymeren: Die Abweichungen bei der Viskosität (üblicherweise eine Viskositätsabnahme) fallen deutlich geringer aus und sind vernachlässigbar klein. Ebenso ist die Gelkonsistenz (d.h. die Textur des Geles, also ob sich beispielsweise Strukturen im Gel finden oder nicht) besser (das heisst die Textur ist geringer oder es ist gar keine Textur vorhanden, das Gel also durchgängig glatt und konturlos) auch nach längerem Lagern. Die beobachtete Stabilitäten der Viskositäten lagen bei gleichen Messbedingungen um bis zu 10 Prozent, bevorzugt bis zu 20 Prozent, besonders bevorzugt bis zu 30 Prozent, ganz besonders bevorzugt bis zu 40 Prozent und insbesondere bis zu 50 Prozent über denen der bisherigen Polymeren.

Ebenso wurde beobachtet, dass die Polymere, insbesondere bei PVP, das gemäß einer der bevorzugten Ausführungsformen erhalten wurde, und damit auch deren Formulierungen wie Haargele geruchlich deutlich besser bewertet werden als bisherige Polymere: Der Geruch der Polymere wie der Haargele ist frischer und neutral bis zu einem ganz schwachen Eigengeruch. Bisher beobachteter stärkerer Eigengeruch oder gar muffige und maismehl-artige, dumpfe Gerüche werden mit den erfindungsgemäßen Polymeren nicht beobachtet.

Weiterhin liegt der pH-Wert der erhältlichen Polymere, insbesondere bei PVP, das gemäß einer der bevorzugten Ausführungsformen erhalten wurde, höher als bei bisherigen Polymeren, weshalb die zur Neutralisation nötige Basenmenge geringer ausfällt und die Polymere und damit die Formulierungen durch weniger Zusatzstoff wie Base belastet werden.

Ebenso wurde festgestellt, dass die Haarfestigung bei Haargelen, insbesondere solche des Carbomer-Typs, bei erfindungsgemäßen Polymeren, insbesondere bei PVP, das gemäß einer der bevorzugten Ausführungsformen 1 bis 84 erhalten wurde und insbesondere einen K-Wert zwischen 20 und 35 aufweist, bis zu 10 Prozent, bevorzugt bis zu 20 Prozent, besonders bevorzugt bis zu 30 Prozent, ganz besonders bevorzugt bis zu 40 Prozent und beispielsweise bis zu 50 Prozent besser ist als bei bisherigen Polymeren. Ein "besseres" Ergebnis bedeutet, dass gleiche Festigungswirkung erreicht werden kann mit entsprechend weniger Polymer, wodurch eine Kosteneinsparung und größere Freiheiten bei der Formulierung möglich werden. Alternativ kann natürlich bei gleicher Menge an Polymer auch eine höhere Festigungswirkung erreicht werden.

Überraschenderweise waren die Polymerpulver, insbesondere bei PVP die mittels Wasserstoffperoxid polymerisiert wurden und insbesondere einen K-Wert zwischen 20 und 35 aufweisen, weniger staubend bei vergleichbarer Fließfähigkeit trotz Trocknung mittels Sprühtrocknung im gleichen Trockner bei gleichen Bedingungen.

Die Bestimmung der Staubfähigkeit wurde wie folgt durchgeführt: eine Glasflasche von 250 ml Volumen wurde zu 2/3 mit Polymerpulver gefüllt und verschlossen. Die Flasche wurde für eine halbe Minute mit der Hand mehrmals in alle Richtungen geschüttelt, abgestellt und dann der Deckel geöffnet: Waren keine Staubwolken oder "Rauch" zu sehen, der aus der offenen Flasche herauskam, so wurde das Polymerpulver als "nicht staubend" bezeichnet. Bildeten sich minimale Schwaden, wurde das Pulver als "minimal staubend" klassifiziert. Entsprechend stärkeres Stauben bedeuten die Bezeichnungen "kaum staubend", "leicht staubend", "staubend" in entsprechend steigender Reihenfolge.

Diese Fähigkeit zur Staubbildung ist insbesondere relevant für das praktische Arbeiten mit solchen Polymerpulvern, etwa beim Umfüllen und Formulieren.

Polymer erhältlich nach dem erfindungsgemäßen Verfahren wie insbesondere nach einer der Ausführungsformen 1 bis 84 ist bevorzugt kaum staubend, besonders bevorzugt minimal staubend und ganz besonders bevorzugt nicht staubend.

### Beispiele

### Bestimmungsmethoden

Generell werden die Methoden zu Festgehalt, pH-Wert, K-Wert, Vinylpyrrolidon und SulfatAsche in der Monographie "Povidone" in der USP und der Ph.Eur. beschrieben. Ameisensäure wird allgemein in der Ph.Eur unter residual solvents (class 3) genannt. Die HAZEN Farbzahl wird alternativ auch als Platin-Kobalt-Farbzahl bezeichnet und in der DIN ISO 6271-1 beschrieben.

Für die vorliegenden Beispiele wurden diese Bestimmungsmethoden angewandt:

| | |
|---|---|
| Festgehalt | 30 Minuten trocknen im Umluftofen bei 140° C, Differenzwiegung ergibt den Festgehalt |
| pH-Wert (10%ig in Wasser) | Ph.Eur.7.Edition, 2.2.3, |
| K-Wert (1%ig (m/V) in Wasser) | nach Fikentscher gemäß Ph.Eur. 7 |
| FTU (10% in Wasser) | nach DIN IS015715 |
| Vinylpyrrolidon | nach Ph.Eu.7 |
| Hazen-Farbzahl | DIN ISO 6271-1 |
| Ameisensäure | enzymatisch |

### Beispiel 1

Es wurden zunächst die folgenden Lösungen vorbereitet:
1. Vorlage, bestehend aus 1688 g vollentsalztem Wasser, 750 g N-Vinylpyrrolidon und 5,58 g Ammoniakwasser (25 %ig)
2. Zulauf 1, bestehend aus 8,81 g Wasserstoffperoxid (50 %ig)
3. Zulauf 2, bestehend aus 0,081 g Kupferchlorid-Lösung (0,091 %ig)
4. Zulauf 3, bestehend aus 61,5 g schwefelige Säure (6 %ig)
5. Zulauf 4, bestehend aus 1,9 g Ammoniak (25 %ig)

In einer Laborapparatur aus Glas mit Rückflusskühler und Halbmondrührer wurde unter einem leichten Stickstoffstrom und unter Rühren die Vorlage auf 75° C aufgeheizt. Bei Erreichen der Temperatur von 75° C erfolgte die Zugabe von Zulauf 1 in weniger als 1 Minute und unmittelbar danach die Zugabe von Zulauf 2. Kurz darauf wurde eine Wärmetönung der Polymerlösung beobachtet, die nach 6 Minuten bis auf 100 ° C bei leichtem Rückfluss anstieg. Danach wurde die Lösung noch 1 h bei 85 ° C weitergerührt. Der pH-Wert lag vor H2O2 Zugabe zunächst bei 10 und am Ende der Polymerisation bei 7,2. Anschließend wurde die Polymerlösung mit Zulauf 3 auf einen pH-Wert von ca. 3,6 unter Rühren eingestellt. Die Lösung wurde erneut auf ca. 100 ° C erhitzt und 1 h mit Wasserdampf gestrippt. Dabei wurde eine Destillatmenge von 500 g er-halten. Nach der Wasserdampfstrippung wurde die Lösung abgekühlt und mit Zulauf 4 neutrali-siert.

Es wurde eine nahezu farblose transparente Polymerlösung 1 erhalten mit folgenden Parame-tern:

| | |
|---|---|
| FG (30 min. 140° C): | 30,6 Gew. % |
| pH-Wert (10%ig in Wasser) | 6 |
| K-Wert (1%ig (m/V) in Wasser) | 29,6 |
| FTU (10% in Wasser) nach DIN ISO15715 | 0,75 |
| Vinylpyrrolidon | < 1 ppm |
| Hazen tel qel. | 18 |
| Hazen (3,5 %ige Lösung) | 5 |
| Ameisensäure (Lösung) | 220 ppm |
| Ameisensäure (bezogen auf Polymerisat) | 740 ppm |

### Beispiel 1a (Vergleichsbeispiel)

Der Versuch aus Beipiel 1 wurde wiederholt, aber statt schwefeliger Säure wurde der pH-Wert kleiner 4 mit Salzsäure eingestellt.

Es wurde eine deutlich gelb gefärbte Polymerlösung 1a erhalten mit folgenden Parametern:

| | |
|---|---|
| FG (30 min. 140° C) | 30,7 Gew. % |
| pH-Wert (10%ig in Wasser) | 6,1 |
| K-Wert (1%ig (m/V) in Wasser) | 29,8 |
| FTU (10% in Wasser) nach DIN ISO15715 | 0,73 |
| Vinylpyrrolidon | < 1 ppm |
| Hazen tel quel. | 440 |
| Hazen (3,5 %ige Lösung) | 35 |
| Ameisensäure (Lösung) | 210 ppm |
| Ameisensäure (bezogen auf Polymerisat) | 700 ppm |

### Beispiel 2

Es wurden zunächst die folgenden Lösungen vorbereitet:
1. Vorlage, bestehend aus 1294 g vollentsalztem Wasser, 1125 g N-Vinylpyrrolidon und 8,37 g Ammoniakwasser (25 %ig)
2. Zulauf 1, bestehend aus 13,2 g Wasserstoffperoxid (50 %ig)
3. Zulauf 2, bestehend aus 0,121 g Kupferchlorid-Lösung (0,091 %ig)
4. Zulauf 3, bestehend aus 68,1 g schwefelige Säure (6 %ig)
5. Zulauf 4, bestehend aus 5,4 g Ammoniak (25 %ig)

In einer Laborapparatur aus Glas mit Rückflusskühler und Halbmondrührer wurde unter einem leichten Stickstoffstrom und unter Rühren die Vorlage auf 75° C aufgeheizt. Bei Erreichen der Temperatur von 75° C erfolgte die Zugabe von Zulauf 1 in weniger als 1 Minute und unmittelbar danach die Zugabe von Zulauf 2. Kurz darauf wurde eine heftige Wärmetönung der Polymerlö-sung beobachtet, die nach 3 Minuten bis auf 100 ° C bei starkem Rückfluss anstieg. Danach wurde die Lösung noch 1 h bei 85 ° C weitergerührt. Der pH-Wert lag vor H2O2 Zugabe zu-nächst bei 10,6 und am Ende der Polymerisation bei 6,2. Anschließend wurde die Polymerlö-sung mit Zulauf 3 auf einen pH-Wert von ca. 3,8 unter Rühren eingestellt. Die Lösung wurde erneut auf ca. 100 ° C erhitzt und 45 Min mit Wasserdamppf gestrippt. Dabei wurde eine Destil-latmenge von 200 g erhalten. Nach der Wasserdampfstrippung wurde die Lösung abgekühlt und mit Zulauf 4 neutralisiert.

Es wurde eine nahezu farblose transparente Polymerlösung 2 erhalten mit folgenden Parame-tern:

| | |
|---|---|
| FG (30 min. 140° C) | 44,2 Gew. % |
| pH-Wert (10%ig in Wasser) | 7,4 |
| K-Wert (1%ig (m/V) in Wasser) | 29,4 |
| FTU (10% in Wasser) nach DIN ISO15715 | 0,54 |
| Vinylpyrrolidon | < 1 ppm |
| Hazen tel quel. | 30 |
| Hazen (3,5 %ige Lösung) | 5 |
| Ameisensäure (Lösung) | 365 ppm |
| Ameisensäure (bezogen auf Polymerisat) | 830 ppm |

### Beispiel 2a (Vergleichsbeispiel)

Der Versuch aus Beispiel 2 wurde wiederholt, aber statt schwefeliger Säure wurde der pH-Wert kleiner 4 mit Schwefelsäure eingestellt.

Es wurde eine deutlich gelb gefärbte Polymerlösung 2a erhalten mit folgenden Parametern:

| | |
|---|---|
| FG (30 min. 140° C) | 44,0 Gew. % |
| pH-Wert (10%ig in Wasser) | 7,8 |
| K-Wert (1%ig (m/V) in Wasser) | 29,7 |
| FTU (10% in Wasser) nach DIN ISO15715 | 0,73 |
| Vinylpyrrolidon | < 1 ppm |
| Hazen tel quel. | 580 |
| Hazen (3,5 %ige Lösung) | 37 |
| Ameisensäure (Lösung) | 460 ppm |
| Ameisenäure (bezogen auf Polymerisat) | 1050 ppm |

### Beispiel 3

Es wurden zunächst die folgenden Lösungen vorbereitet:
1. Vorlage, bestehend aus 1294 g vollentsalztem Wasser, 1125 g N-Vinylpyrrolidon und 8,37 g Ammoniakwasser (25 %ig)
2. Zulauf 1, bestehend aus 17,8 g Wasserstoffperoxid (50 %ig)
3. Zulauf 2, bestehend aus 0,121 g Kupferchlorid-Lösung (0,091 %ig)
4. Zulauf 3, bestehend aus 60,4 g schwefelige Säure (6 %ig)
5. Zulauf 4, bestehend aus 4,8 g Ammoniak (25 %ig)

In einer Laborapparatur aus Glas mit Rückflusskühler und Halbmondrührer wurde unter einem leichten Stickstoffstrom und unter Rühren die Vorlage auf 73° C aufgeheizt. Bei Erreichen der Temperatur von 73° C erfolgte die Zugabe von Zulauf 1 in weniger als 1 Minute und unmittelbar danach die Zugabe von Zulauf 2. Kurz darauf wurde eine heftige Wärmetönung der Polymerlö-sung beobachtet, die nach 4 Minuten bis auf 100 ° C bei starkem Rückfluss anstieg. Danach wurde die Lösung noch 1 h bei 85 ° C weitergerührt. Der pH-Wert lag vor H2O2 Zugabe zu-nächst bei 10,8 und am Ende der Polymerisation bei 5,0. Anschließend wurde die Polymerlösung mit Zulauf 3 auf einen pH-Wert von ca. 3,8 unter Rühren eingestellt. Die Lösung wurde erneut auf ca. 100 ° C erhitzt und 1 h mit Wasserdamppf gestrippt. Dabei wurde eine Destillatmenge von 200 g erhalten. Nach der Wasserdampfstrippung wurde die Lösung abge-kühlt und mit Zulauf 4 neutralisiert.

Es wurde eine nahezu farblose transparente Polymerlösung 3 erhalten mit folgenden Parame-tern:

| | |
|---|---|
| FG (30 min. 140° C) | 43,6 Gew. % |
| pH-Wert (10%ig in Wasser) | 6,8 |
| K-Wert (1%ig (m/V) in Wasser) | 24,5 |
| FTU (10% in Wasser) nach DIN ISO15715 | 0,52 |
| Vinylpyrrolidon | < 1 ppm |
| Hazen tel quel. | 75 |
| Hazen (3,5 %ige Lösung) | 6 |
| Ameisensäure (Lösung) | 445 ppm |
| Ameisensäure (bezogen auf Polymerisat) | 1020 ppm |

### Beispiel 3a (Vergleichsbeispiel)

Der Versuch aus Beispiel 5 wurde wiederholt, aber statt schwefeliger Säure wurde der pH-Wert kleiner 4 mit Schwefelsäure eingestellt.

Es wurde eine deutlich gelblich gefärbte Polymerlösung 3a erhalten mit folgenden Parametern:

| | |
|---|---|
| FG (30 min. 140° C) | 43,7 Gew. % |
| pH-Wert (10%ig in Wasser) | 6,8 |
| K-Wert (1%ig (m/V) in Wasser) | 24,4 |
| FTU (10% in Wasser) nach DIN ISO 15715 | 0,55 |
| Vinylpyrrolidon | < 1 ppm |
| Hazen tel qel. | 620 |
| Hazen (3,5 %ige Lösung) | 41 |
| Ameisensäure (Lösung) | 450 ppm |
| Ameisensäure (bezogen auf Polymerisat) | 1030 ppm |

### Beispiel 4

Es wurden zunächst die folgenden Lösungen vorbereitet:
1. Vorlage, bestehend aus 1640 g vollentsalztem Wasser, 800 g N-Vinylpyrrolidon und 2,7 g Ammoniumhydrogencarbonat
2. Zulauf 1, bestehend aus 9,4 g Wasserstoffperoxid (50 %ig)
3. Zulauf 2, bestehend aus 0,086 g Kupferchlorid-Lösung (0,091 %ig)
4. Zulauf 3, bestehend aus 34,3 g schwefelige Säure (6 %ig)
5. Zulauf 4, bestehend aus 1,9 g Ammoniak (25 %ig)

In einer Laborapparatur aus Glas mit Rückflusskühler und Halbmondrührer wurde unter einem leichten Stickstoffstrom und unter Rühren die Vorlage auf 74° C aufgeheizt. Bei Erreichen der Temperatur von 74° C erfolgte die Zugabe von Zulauf 1 in weniger als 1 Minute und unmittelbar danach die Zugabe von Zulauf 2. Kurz darauf wurde eine Wärmetönung der Polymerlösung beobachtet, die nach 10 Minuten bis auf 100 ° C bei leichtem Rückfluss anstieg. Danach wurde die Lösung noch 1 h bei 85 ° C weitergerührt. Der pH-Wert lag vor H2O2 Zugabe zunächst bei 8,0 und am Ende der Polymerisation bei 6,2. Anschließend wurde die Polymerlösung mit Zu-lauf 3 auf einen pH-Wert von ca. 3,7 unter Rühren eingestellt. Die Lösung wurde erneut auf ca. 100 ° C erhitzt und 1 h mit Wasserdampf gestrippt. Dabei wurde eine Destillatmenge von 450 g erhalten. Nach der Wasserdampfstrippung wurde die Lösung abgekühlt und mit Zulauf 4 neutralisiert.

Es wurde eine nahezu farblose transparente Polymerlösung 4 erhalten mit folgenden Parame-tern:

| | |
|---|---|
| FG (30 min. 140° C) | 31,7 Gew. % |
| pH-Wert (10%ig in Wasser) | 7,7 |
| K-Wert (1%ig (m/V) in Wasser) | 28,4 |
| FTU (10% in Wasser) nach DIN ISO15715 | 0,49 |
| Vinylpyrrolidon | < 1 ppm |
| Hazen tel quel. | 20 |
| Hazen (3,5 %ige Lösung) | 5 |
| Ameisensäure (Lösung) | 230 ppm |
| Ameisensäure (bezogen auf Polymerisat) | 730 ppm |

### Beispiel 5

Es wurden zunächst die folgenden Lösungen vorbereitet:
1. Vorlage, bestehend aus 1688 g vollentsalztem Wasser, 750 g N-Vinylpyrrolidon und 2,5 g Natriumcarbonat
2. Zulauf 1, bestehend aus 8,8 g Wasserstoffperoxid (50 %ig)
3. Zulauf 2, bestehend aus 0,081 g Kupferchlorid-Lösung (0,091 %ig)
4. Zulauf 3, bestehend aus 39,9 g schwefelige Säure (6 %ig)
5. Zulauf 4, bestehend aus 2,2 g Ammoniak (25 %ig)

In einer Laborapparatur aus Glas mit Rückflusskühler und Halbmondrührer wurde unter einem leichten Stickstoffstrom und unter Rühren die Vorlage auf 73° C aufgeheizt. Bei Erreichen der Temperatur von 73° C erfolgte die Zugabe von Zulauf 1 in weniger als 1 Minute und unmittelbar danach die Zugabe von Zulauf 2. Kurz darauf wurde eine Wärmetönung der Polymerlösung beobachtet, die nach 10 Minuten bis auf 100 ° C bei leichtem Rückfluss anstieg. Danach wurde die Lösung noch 1 h bei 85 ° C weitergerührt. Der pH-Wert lag vor H2O2 Zugabe zunächst bei 11,2 und am Ende der Polymerisation bei 6,8. Anschließend wurde die Polymerlösung mit Zulauf 3 auf einen pH-Wert von ca. 3,9 unter Rühren eingestellt. Die Lösung wurde erneut auf ca. 100 ° C erhitzt und 1 h mit Wasserdamppf gestrippt. Dabei wurde eine Destillatmenge von 260 g erhalten. Nach der Wasserdampfstrippung wurde die Lösung abgekühlt und mit Zulauf 4 neutralisiert.

Es wurde eine nahezu farblose transparente Polymerlösung 5 erhalten mit folgenden Parame-tern:

| | |
|---|---|
| FG (30 min. 140° C) | 30,0 Gew. % |
| pH-Wert (10%ig in Wasser) | 5,9 |
| K-Wert (1%ig (m/V) in Wasser) | 32,2 |
| FTU (10% in Wasser) nach DIN ISO15715 | 0,48 |
| Vinylpyrrolidon | 4 ppm |
| Hazen tel quel. | 18 |
| Hazen (3,5 %ige Lösung) | 5 |
| Ameisensäure (Lösung) | 210 ppm |
| Ameisensäure (bezogen auf Polymerisat) | 700 ppm |

### Beispiel 6

Es wurden zunächst die folgenden Lösungen vorbereitet:
1. Vorlage, bestehend aus 1737 g vollentsalztem Wasser, 1000 g N-Vinylpyrrolidon und 3,72 g Ammoniakwasser (25 %ig)
2. Zulauf 1, bestehend aus 10,6 g Wasserstoffperoxid (50 %ig)
3. Zulauf 2, bestehend aus 0,107 g Kupferchlorid-Lösung (0,091 %ig)
4. Zulauf 3, bestehend aus 55,6 g schwefelige Säure (6 %ig)
5. Zulauf 4, bestehend aus 3,5 g Ammoniak (25 %ig)

In einer Laborapparatur aus Glas mit Rückflusskühler und Halbmondrührer wurde unter einem leichten Stickstoffstrom und unter Rühren die Vorlage auf 50° C aufgeheizt. Bei Erreichen der Temperatur von 50 ° C erfolgte die Zugabe von Zulauf 1 in weniger als 1 Minute und unmittelbar danach die Zugabe von Zulauf 2. Kurz darauf wurde eine Wärmetönung der Polymerlösung beobachtet, die nach 15 Minuten bis auf 100 ° C bei mäßigem Rückfluss anstieg. Danach wurde die Lösung noch 1 h bei 80 ° C weitergerührt. Anschließend wurde die Polymerlösung mit Zulauf 3 auf einen pH-Wert von ca. 3,6 unter Rühren eingestellt. Die Lösung wurde erneut auf ca. 100 ° C erhitzt und 1 h mit Wasserdamppf gestrippt. Dabei wurde eine Destillatmenge von 400 g erhalten. Nach der Wasserdampfstrippung wurde die Lösung abgekühlt und mit Zulauf 4 neutralisiert und mit Wasser auf einen Feststoffgehalt von 30 % eingestellt.

Es wurde eine nahezu farblose Polymerlösung 6 erhalten mit folgenden Parametern:

| | |
|---|---|
| FG (30 min. 140° C) | 29,8 Gew. % |
| pH-Wert (10%ig in Wasser) | 8,4 |
| K-Wert (1%ig (m/V) in Wasser) | 32,3 |
| FTU (10% in Wasser) nach DIN ISO15715 | 0,44 |
| Vinylpyrrolidon | < 1 ppm |
| Hazen tel quel. | 19 |
| Hazen (3,5 %ige Lösung) | 6 |
| Ameisensäure (Lösung) | 225 ppm |
| Ameisensäure (bezogen auf Polymerisat) | 750 ppm |

Entsprechend den Angaben der DE 11 2005 002 719 wurden die Polymerisate der Beispiele 1, 2 und 3 hergestellt und analysiert:

| | |
|---|---|
| | Beispiele 1 / 2 / 3 |
| Feststoffgehalt | 49,1 / 49,1 / 49,2 |
| K-Wert | 23 / 23,5 / 25,8 |
| HAZEN (tel quel. | 180 / 210 / 160 |
| HAZEN (3,5 %ige Lsg) | 12 / 15 / 10 |
| Ameisensäure Lösung | 4000 / 3700 / 3600 ppm |
| Ameisensäure (bezogen auf Polymerisat) | 8150 / 7550 / 7300 ppm |

Die erfindungsgemäßen Polymerlösungen 1 - 6 wurden zusammen mit den entsprechend der Veröffentlichung DE 11 2005 002 719 hergestellten Polymerlösungen Beispiel 1 bis 3 drei Monate bei 40 ° C gelagert und der HAZEN Farbwert nach 1, 2 und 3 Monaten an einer 3,5 %igen Lösung gemessen (Tabelle 1)

**Tabelle 1: Beispiele 1 bis 6, Vergleichsbeispiele aus DE 11 2005 002 719**

| HAZEN-Farbzahl / Polymerlösung | Startwert | HAZEN-Farbzahl nach 1 Monat | HAZEN-Farbzahl nach 2 Monaten | HAZEN-Farbzahl nach 3 Monaten | Bemerkung |
|---|---|---|---|---|---|
| 1 | 5 | 5 | 6 | 6 | 1 |
| 2 | 5 | 6 | 6 | 7 | 1 |
| 3 | 6 | 5 | 6 | 6 | 1 |
| 4 | 5 | 6 | 6 | 7 | 1 |
| 5 | 5 | 5 | 5 | 6 | 1 |
| 6 | 5 | 5 | 6 | 6 | 1 |
| Beispiel 1 (DE) | 12 | 15 | 20 | 26 | 2 |
| Beispiel 2 (DE) | 15 | 17 | 18 | 30 | 2 |
| Beispiel 3 (DE) | 10 | 15 | 20 | 24 | 2 |

| | | | | | |
|---|---|---|---|---|---|
| Bemerkungen 1: keine farbliche Veränderung Bemerkungen 2: Starke Verfärbung | | | | | |

Wässrige Polymerlösungen, erhalten nach erfindungsgemäßen Beispielen 3 und 4 und einer Lösung erhalten nach Vergleichsbeispiel 3a

| Geruch, Klarheit und Farbe - bei Polymerfestgehalt wie erhalten | | | |
|---|---|---|---|
| Polymer | nach Ansatz | nach 4 Wochen Lagerung bei | |
| | | 25° C | 40° C |
| Lösung aus Beispiel 3a | klar, gelb nicht o.k. (Farbe) Geruch: wenig intensiv, minimal terpenartig, minimal süsslich, nicht dumpf; o.k. Gesamturteil: nicht o.k. (Farbe) | gelb, klar nicht o.k. (Farbe) Geruch: wenig intensiv, minimal terpenartig, o.k. Gesamturteil: nicht o.k. (Farbe) | intensiv gelb, klar nicht o.k. (Farbe) Geruch: wenig intensiv, minimal terpenartig, minimal maismehlartig; noch o.k. Gesamturteil: nicht o.k. (Farbe) |
| Lösung aus Beispiel 3 | farblos, nahezu klar o.k. Geruch: wenig intensiv, frisch, minimal terpenartig, minimal Trockenhefe, besser als Standard; o.k. Gesamturteil: o.k. | nahezu klar, farblos o.k. Geruch: wenig intensiv, minimal trockenhefeartig, minimal terpenartig, angenehm; o.k. Gesamturteil: o.k. | klar, nahezu farblos; o.k. Geruch: wenig intensiv, minimal terpenartig, minimal trockenhefeartig o.k. Gesamturteil: o.k. |
| Lösung aus Beispiel 4 | farblos, klar; o.k. Geruch: wenig intensiv, minimal terpenartig, minimal Trockenhefe, angenehm, besser als Standard; o.k. Gesamturteil: o.k. | klar/nahezu klar, farblos o.k. Geruch: wenig intensiv, minimal trockenhefeartig, minimal terpenartig; o.k. Gesamturteil: o.k. | nahezu farblos, nahezu klar o.k-noch o.k. (Farbe) Geruch: wenig intensiv, minimal trockenhefeartig, minimal terpenartig; o.k. Gesamturteil: o.k.-noch o.k. (Farbe) |

Wässrige Polymerlösungen, erhalten nach erfindungsgemäßen Beispielen 3 und 4 und einer Lösung erhalten nach Vergleichsbeispiel 3a

| Geruch, Klarheit und Farbe - bei 5 Gew.% Polymerfestgehalt | | | |
|---|---|---|---|
| Polymer | nach Ansatz | nach 4 Wochen Lagerung bei | |
| | | 25° C | 40° C |
| Lösung aus Beispiel 3a | klar, minimalst gelblich noch o.k. Geruch: wenig intensiv, nicht dumpf, leicht blumig, frisch, angenehm o.k. Gesamturteil: noch o.k. | minimal gelblich, klar Grenzfall-nicht o.k. (Farbe) Geruch: wenig intensiv, frisch, leicht terpenartig o.k. Gesamturteil: nicht o.k. (Farbe) | leicht gelblich-gelblich, klar; nicht o.k. (Farbe) Geruch: wenig intensiv, minimal terpenartig, minimal blumig, nicht dumpf, kein Maismehl o.k. Gesamturteil: nicht o.k. (Farbe) |
| Lösung aus Beispiel 3 | klar, farblos o.k. Geruch: wenig intensiv, minimal Trockenhefe, angenehm, weniger intensiv/besser als Standard o.k. Gesamturteil: o.k. | klar, farblos o.k. Geruch: wenig intensiv, minimal blumig, terpenartig, frisch; o.k. Gesamturteil: o.k. | klar, farblos o.k. Geruch: wenig intensiv, minimal terpenartig, nicht dumpf, kein Maismehl, frisch; o.k. Gesamturteil: o.k. |
| Lösung aus Beispiel 4 | klar, farblos; o.k. Geruch: nahezu geruchlos, minimaler Trockenhefegeruch, angenehm, weniger intensiv/besser als Standard; o.k. Gesamturteil: o.k. | klar, farblos; o.k. Geruch: wenig intensiv, minimal terpenartig, frisch, ähnlich Standard o.k. Gesamturteil: o.k. | klar, farblos; o.k. Geruch: wenig intensiv, minimal terpenartig, nicht dumpf, kein Maismehl o.k. Gesamturteil: o.k. |

Wässrige Polymerlösungen, erhalten nach erfindungsgemäßen Beispielen 3 und 4 und einer Lösung erhalten nach Vergleichsbeispiel 3a

| pH-Wert - bei Polymerfestgehalt wie erhalten | | | |
|---|---|---|---|
| Polymer | nach Ansatz | nach 4 Wochen Lagerung bei | |
| | | 25° C | 40° C |
| Lösung aus Beispiel 3a | 4,7 | 4,7 | 4,6 |
| Lösung aus Beispiel 3 | 7,25 | 7,7 | 7,3 |
| Lösung aus Beispiel 4 | 7,5 | 7,9 | 7,75 |

| Haar-Gele aus Polymerlösungen | | | |
|---|---|---|---|
| PVP K 30 | nach Ansatz | nach 4 Wochen Lagerung bei | |
| | | 25° C | 40° C |
| Lösung aus Beispiel 3a | minimal gelblich, leicht blaustichig, festes Gel nicht o.k. (Farbe) Geruch: typisch Carbopol; o.k. Gesamturteil: nicht o.k. (Farbe) | leicht gelblich, minimal blaustichig, noch festes Gel; nicht o.k. (Farbe) Geruch: wenig intensiv, typisch Carbopol; o.k. Gesamturteil: nicht o.k. (Farbe) | gelb, minimal blaustichig, noch festes Gel; nicht o.k. (Farbe) Geruch: intensiver Carbopolgeruch; noch o.k. Gesamturteil: nicht o.k. (Farbe) |
| Lösung aus Beispiel 3 | farblos, leicht blaustichig, festes Gel; o.k. Geruch: wenig intensiv, typisch Carbopol; o.k. Gesamturteil: o.k. | absolut farblos, leicht blaustichig, festes Gel o.k. Geruch: wenig intensiv, typisch Carbopol; o.k. Gesamturteil: o.k. | farblos, minimal blaustichig, festes Gel; o.k. wenig intensiv, typisch Carbopol; o.k. Gesamturteil: o.k. |
| Lösung aus Beispiel 4 | farblos, leicht blaustichig, festes Gel; o.k. Geruch: wenig intensiv, typisch Carbopol; o.k. Gesamturteil: o.k. | farblos, minimal blaustichig, festes Gel; o.k. Geruch: wenig intensiv, typisch Carbopol; o.k. Gesamturteil: o.k. | farblos, leicht blaustichig, festes Gel; o.k. Geruch: wenig intensiv, typisch Carbopol; o.k. Gesamturteil: o.k. |

5% Polymergehalt. in Carbopol 980 (0,5%). Alle Versuche sind mit dem gleichen Grundgel-Rezept hergestellt. Grundgel mit Triethylamin auf pH 7 eingestellt, mit Euxyl K 100 konserviert.

### Haar-Gele aus Polymerlösungen. Stabilität beim Lagern

5% Polymergehalt. in Carbopol 980 (0,5%). Alle Versuche sind mit dem gleichen Grundgel-Rezept hergestellt. Grundgel mit Triethylamin auf pH 7 eingestellt, mit Euxyl K 100 konserviert.

| Polymer | nach Ansatz | | | |
|---|---|---|---|---|
| | Biegetest Mittelwert [cN]** | Curl retention [%] | Viskosität [mPas] | pH-Wert |
| Lösung aus Beispiel 3a | 62 (58-67) | 37 | 26700 | 6,5 |
| Lösung aus Beispiel 3 | 87 (81-92) | 39 | 23800 | 6,65 |
| Lösung aus Beispiel 4 | 83 (79-87) | 34 | 25050 | 6,65 |

| | | | | |
|---|---|---|---|---|
| ** Angaben in Klammern : Messwertbereich von mind. 5 Strähnen | | | | |

| Polymer | nach 4 Wochen | | | |
|---|---|---|---|---|
| | Viskosität [mPas] | | pH-Wert | |
| | RT | 40° C | RT | 40° C |
| Lösung aus Beispiel 3a | 23100 | 21650 | 6,5 | 6,5 |
| Lösung aus Beispiel 3 | 22500 | 23000 | 6,6 | 6,6 |
| Lösung aus Beispiel 4 | 24500 | 24850 | 6,7 | 6,6 |

Wässrige Polymerlösungen, erhalten nach erfindungsgemäßen Beispielen 3 und 4 und einer Lösung erhalten nach Vergleichsbeispiel 3a, nach Trocknung zum Pulver mittels Sprühtrocknung in Luft.

| Geruch, Farbe, Staubfähigkeit, Rieselfähigkeit | | | |
|---|---|---|---|
| Polymer | nach Ansatz | nach 4 Wochen Lagerung bei | |
| | | 25° C | 40° C |
| Polymer aus Beispiel 3a | minimalst gelblich, kristallines, gut fließfähiges Pulver, staubt; noch o.k. Geruch: nahezu geruchlos; o.k. Gesamturteil: noch o.k. | nahezu weiss-minimal cremefarben, gut fließfähig, minimal staubend; o.k. Geruch: nahezu geruchlos, minimal dumpf; o.k. Gesamturteil: o.k. | cremefarben-minimalst gelblich, gut fließfähig, leicht staubend-staubend; noch o.k. (Farbe) Geruch: nahezu geruchlos; o.k. Gesamturteil: noch o.k. (Farbe) |
| Polymer aus Beispiel 3 | weißes, kristallines, gut fließfähiges Pulver, leicht staubend o.k.; Geruch: nahezu geruchlos; o.k. Gesamturteil: o.k. | nahezu weiss, gut fließfähig, vgl. Standard, kaum staubend; o.k. Geruch: wenig intensiv, minimal dumpf/ maismehlartig, noch o.k. Gesamturteil: o.k. | nahezu weiss-minimal cremefarben, besser als Standard, gut fließfähig, kaum staubend o.k. Geruch: nahezu geruchlos; o.k. Gesamturteil: o.k. |
| Polymer aus Beispiel 4 | weißes, kristallines, gut fließfähiges Pulver, leicht staubend o.k. Geruch: nahezu geruchlos; o.k. Gesamturteil: o.k. | nahezu weiss, gut fließfähig, vgl. Standard, minimal staubend; o.k. Geruch: wenig intensiv, kaum dumpf; o.k. Gesamturteil: o.k. | nahezu weiss-minimal cremefarben, gut fließfähig, minimal staubend; o.k. Geruch: nahezu geruchlos; o.k. Gesamturteil: o.k. |

Wässrige Polymerlösungen, erhalten nach erfindungsgemäßen Beispielen 3 und 4 und einer Lösung erhalten nach Vergleichsbeispiel 3a, nach Trocknung zum Pulver mittels Sprühtrocknung in Luft und Wiederauflösen des Pulvers zu einer wässrigen Lösung

| Geruch, Klarheit und Farbe - bei 5 Gew.% Polymerfestgehalt | | | |
|---|---|---|---|
| Polymer | nach Ansatz | nach 4 Wochen Lagerung bei | |
| | | 25° C | 40° C |
| Polymer aus Beispiel 3a | minimalst gelblich (besser als Standard Lösung), klar; noch o.k. Geruch: nahezu geruchlos, minimal terpenartig, frisch, angenehm; o.k. Gesamturteil: noch o.k. | minimal gelblich, klar Grenzfall-nicht o.k. (Farbe) Geruch: wenig intensiv, minimal terpenartig, minimal blumig; o.k. Gesamturteil: Grenzfall-nicht o.k. (Farbe) | minimal gelblich, vgl. Rt, klar; Grenzfall-nicht o.k. (Farbe) Geruch: wenig intensiv, minimal terpenartig, minimal blumig; o.k. Gesamturteil: Grenzfall-nicht o.k. (Farbe) |
| Polymer aus Beispiel 3 | farblos, klar; o.k. Geruch: wenig intensiv, nicht dumpf, minimal fruchtig, angenehm; o.k. Gesamturteil: o.k. | farblos, klar; o.k. Geruch: wenig intensiv, minimal terpenartig, minimal blumig, leicht süsslich; o.k. Gesamturteil: o.k. | farblos, klar; o.k. Geruch: wenig intensiv, minimal terpenartig, minimal blumig; o.k. Gesamturteil: o.k. |
| Polymer aus Beispiel 4 | farblos, klar; o.k. Geruch: nahezu geruchlos, minimal terpenartig, frisch, angenehm; o.k. Gesamturteil: o.k. | farblos, klar; o.k. Geruch: nahezu geruchlos; o.k. Gesamturteil: o.k. | farblos, klar; o.k. Geruch: nahezu geruchlos; o.k. Gesamturteil: o.k. |

| pH-Wert bei 5 Gew.% Polymerfestgehalt | | | |
|---|---|---|---|
| Polymer | nach Ansatz | nach 4 Wochen Lagerung bei | |
| | | 25° C | 40° C |
| Polymer aus Beispiel 3a | 3,8 | 3,7 | 3,6 |
| Polymer aus Beispiel 3 | 3,8 | 3,5 | 3,45 |
| Polymer aus Beispiel 4 | 3,45 | 3,2 | 3,2 |

Haar-Gele aus Pulvern - Stabilität beim Lagern Wässrige Polymerlösungen, erhalten nach erfindungsgemäßen Beispielen 3 und 4 und einer Lösung erhalten nach Vergleichsbeispiel 3a, nach Trocknung zum Pulver mittels Sprühtrocknung in Luft und Wiederauflösen des Pulvers zu einer wässrigen Lösung

5% Polymergehalt. in Carbopol 980 (0,5%). Alle Versuche sind mit dem gleichen Grundgel-Rezept hergestellt. Grundgel mit Triethylamin auf pH 7 eingestellt, mit Euxyl K 100 konserviert.

| Polymer | nach Ansatz | nach 4 Wochen Lagerung bei | |
|---|---|---|---|
| | | 25° C | 40° C |
| Polymer aus Beispiel 3a | minimalst gelblich (weniger gelblich als K30L Standard), blaustichig, noch festes Gel; noch o.k. wenig intensiv, typisch Carbopol; o.k Gesamturteil: noch o.k. | minimal gelblich, leicht blaustichig, leicht verfließendes Gel; nicht o.k. (Visko und Farbe) wenig intensiv, typisch Carbopol ; o.k. Gesamturteil: nicht o.k. (Viskosität, Farbe) | gelblich, leicht blaustichig, leicht verfließendes Gel nicht o.k. (Visko und Farbe) intensiver Carbopolgeruch noch o.k. Gesamturteil: nicht o.k. (Visko, Farbe) |
| Polymer aus Beispiel 3 | farblos, nahezu klarminimal blaustichig, festes Gel; o.k. wenig intensiv, typisch Carbopol; o.k. Gesamturteil: o.k. (besser als Standard in Visko und Trübung) | nahezu farblos/minimalst gelblich, nahezu klar, festes Gel; o.k. wenig intensiv, typisch Carbopol; o.k. Gesamturteil: o.k. | nahezu farblos, minimalst blaustichig, festes Gel o.k.; wenig intensiv, typisch Carbopol; o.k. Gesamturteil: o.k. |
| Polymer aus Beispiel 4 | minimalst gelblich, nahezu klar/minimal blaustichig, noch festes Gel; noch o.k. wenig intensiv, typisch Carbopol; o.k. Gesamturteil: noch o.k. (Farbe) | nahezu farblos/minimalst gelblich, nahezu klar/minimalst blaustichig, festes Gel; o.k.-noch o.k. (Farbe) wenig intensiv, typisch Carbopol; o.k. Gesamturteil: o.k.-noch o.k. (Farbe) | nahezu farblos/minimalst gelblich, nahezu klar/minimalst blaustichig, festes Gel o.k.-noch o.k.(Farbe) wenig intensiv, typisch Carbopol; o.k. Gesamturteil: o.k.-noch o.k. (Farbe) |

| Polymer | nach Ansatz | | | |
|---|---|---|---|---|
| | Biegetest Mittelwert [cN] ** | Curl retention [%] | Viskosität [mPas] | pH-Wert |
| Polymer aus Beispiel 3a | 67 (63-71) | 33 | 19100 | 6,5 |
| Polymer aus Beispiel 3 | 90 (86-95) | 34 | 25350 | 6,55 |
| Polymer aus Beispiel 4 | 91 (88-95) | 33 | 24250 | 6,5 |

| | | | | |
|---|---|---|---|---|
| ** Angaben in Klammern : Messwertbereich von mind. 5 Strähnen | | | | |

| Polymer | nach 4 Wochen | | | |
|---|---|---|---|---|
| | Viskosität [mPas] | | pH-Wert | |
| | RT | 40° C | RT | 40° C |
| Polymer aus Beispiel 3a | 14850 | 13800 | 6,35 | 6,4 |
| Polymer aus Beispiel 3 | 24300 | 25100 | 6,5 | 6,5 |
| Polymer aus Beispiel 4 | 23050 | 23450 | 6,3 | 6,4 |

Vergleich zur DE 10 2005 00 5974 (Beispiel 3 aus der DE 10 2005 00 5974, die dieses Beispiel als Vergleich zu GB 836,831 offenbart).

Eine 20 %ige wässrige Lösung von Polyvinylpyrrolidon mit einem K-Wert von 30 (gemessen in einer 1 Gew.-%igen wässrigen Lösung) wurde bei 80° C mit 0,1 Gew.-% Schwefeldioxid bezogen auf Polymer (in Form einer 6 %Lösung von Schwefeldioxid in Wasser) 10 versetzt und die Lösung eine Stunde gerührt. Anschließend wurde die Lösung auf 40° C abgekühlt und sprühgetrocknet. Das pulverförmige Polyvinylpyrrolidon wurde anschließend in Säcke aus Aluminiumverbundfolie eingeschweißt, wobei vor der Verschweißung der gefüllte Sack zweimal mit Stickstoff geflutet wurde und unter 15 verschiedenen Bedingungen gelagert. Weiterhin wurden auch Proben unter Luft eingeschweißt und anschließend bei 25° C gelagert. Der Peroxid-Gehalt wurde direkt nach Behandlung sowie nach drei und sechs Monaten Lagerung bestimmt.

**Tabelle: Beispiel 3 aus der DE 10 2005 00 5974; Peroxidgehalt in ppm (bezogen auf den Polymerfestgehalt)**

| | Verpackung unter | | |
|---|---|---|---|
| | Luft | Stickstoff | Stickstoff |
| Lagerung bei | 25° C | bei 25° C/ 60% RF | bei 40° C/75% RF |
| Nullwert | <1 | <1 | <1 |
| 3 Monate | 339 | 383 | 390 |
| 6 Monate | 458 | 459 | 354 |

Der Peroxidgehalt ist ein Maß für die Oxidation des Polymeren. Nach bekanntem Fachwissen entsteht bei PVP durch Oxidation aber eine Gelbfärbung. Die hohen Peroxidgehalte der Versuche aus DE 10 2005 00 5974 korrelieren mit hohen Farbwerten. Dies zeigt, dass der Zusatz von Schwefeldioxid alleine nicht zu guten Farbwerten und insbesondere nicht zu guter Lagerstabilität führt.

### Vergleich zur GB 836,831

Gemäß der GB 836,831 wurden die pH-Werte von wässrigen Lösungen von PVP mit K-Wert 30 bestimmt: Die Lösung wies einen Feststoffgehalt von 10 Gewichtsprozent bezogen auf festes Polymer auf. Dann wurde der pH-Wert der Lösung mittels Triethanolamin auf 8,01 eingestellt und diese Lösung mit 0,5 Gewichtsprozent Schwefeldioxid beziehungsweise Natriumhydrogensulfit (bezogen auf festes Polymer) versetzt.

Der pH-Wert bei Verwendung von Schwefeldioxid betrug 7,76, bei Verwendung von Natriumhydrogensulfit 7,68.

Dies zeigt, dass bei den Versuchen gemäß der GB 836,831 praktisch keine Absenkung des pH-Wertes durch Zusatz der Schwefelkomponente erreicht wird. Daher wird durch alleinigen Zusatz der Schwefelkomponente auch keine Absenkung des pH-Wertes erreicht, bei dem eine saure Hydrolyse stattfinden würde.

**Übersicht der erfindungsgemäßen Ausführungsformen**

| Ausführungsform Nr. | Basiert auf Ausführungsform Nr. | Ausgeführte Schritte | | | | | | | | | Base in Schritt b) | Schwefel-komponente in Schritt e) | Verfahren bestehend nur aus den Schritten a) bis h) ? |
|---|---|---|---|---|---|---|---|---|---|---|---|---|---|
| | | a | b | c | d | e | d (1)) | f | g | h | | | |
| A 1 | | x | ο | o | o | x | o | o | o | o | bw | sw | |
| A 2 | | x | x | o | o | x | o | o | o | o | bw | sw | |
| 1 | | x | x | o | x | x | o | o | o | o | bw | sw | |
| 2 | | x | x | o | x | x | o | x | o | o | bw | sw | |
| 3 | | x | x | o | x | x | o | o | x | o | bw | sw | |
| 4 | | x | x | o | x | x | o | o | o | x | bw | sw | |
| 5 | | x | x | n | x | x | o | o | o | o | bw | sw | |
| 6 | | x | x | n | x | x | o | x | x | x | bw | sw | |
| 7 | | x | x | n | x | x | x | x | x | x | bw | sw | |
| 8 | 1 | x | x | o | x | x | o | o | o | o | AHC | sw | |
| 9 | 2 | x | x | o | x | x | o | x | o | o | AHC | sw | |
| 10 | 3 | x | x | o | x | x | o | o | x | o | AHC | sw | |
| 11 | 4 | x | x | o | x | x | o | o | o | x | AHC | sw | |
| 12 | 5 | x | x | n | x | x | o | o | o | o | AHC | sw | |
| 13 | 6 | x | x | n | x | x | o | x | x | x | AHC | sw | |
| 14 | 7 | x | x | n | x | x | x | x | x | x | AHC | sw | |
| 15 | 1 | x | x | o | x | x | o | o | o | o | NH3 | sw | |
| 16 | 2 | x | x | o | x | x | o | x | o | o | NH3 | sw | |
| 17 | 3 | x | x | o | x | x | o | o | x | o | NH3 | sw | |
| 18 | 4 | x | x | o | x | x | o | o | o | x | NH3 | sw | |
| 19 | 5 | x | x | n | x | x | o | o | o | o | NH3 | sw | |
| 20 | 6 | x | x | n | x | x | o | x | x | x | NH3 | sw | |
| 21 | 7 | x | x | n | x | x | x | x | x | x | NH3 | sw | |
| 22 | 1 | x | x | o | x | x | o | o | o | o | bw | SO2 | |
| 23 | 2 | x | x | o | x | x | o | x | o | o | bw | SO2 | |
| 24 | 3 | x | x | o | x | x | o | o | x | o | bw | SO2 | |
| 25 | 4 | x | x | o | x | x | o | o | o | x | bw | SO2 | |
| 26 | 5 | x | x | n | x | x | o | ο | ο | ο | bw | SO2 | |
| 27 | 6 | x | x | n | x | x | o | x | x | x | bw | SO2 | |
| 28 | 7 | x | x | n | x | x | x | x | x | x | bw | SO2 | |
| 29 | 8 | x | x | o | x | x | o | o | o | o | AHC | SO2 | |
| 30 | 9 | x | x | o | x | x | o | x | o | o | AHC | SO2 | |
| 31 | 10 | x | x | o | x | x | o | o | x | o | AHC | SO2 | |
| 32 | 11 | x | x | o | x | x | o | o | o | x | AHC | SO2 | |
| | | Ausgeführte Schritte | | | | | | | | | | | |
| 33 | 12 | x | x | n | x | x | o | ο | ο | ο | AHC | SO2 | |
| 34 | 13 | x | x | n | x | x | ο | x | x | x | AHC | SO2 | |
| 35 | 14 | x | x | n | x | x | x | x | x | x | AHC | SO2 | |
| 36 | 15 | x | x | o | x | x | o | o | o | o | NH3 | SO2 | |
| 37 | 16 | x | x | o | x | x | o | x | o | o | NH3 | SO2 | |
| 38 | 17 | x | x | o | x | x | o | o | x | o | NH3 | SO2 | |
| 39 | 18 | x | x | o | x | x | o | o | o | x | NH3 | SO2 | |
| 40 | 19 | x | x | n | x | x | ο | ο | ο | ο | NH3 | SO2 | |
| 41 | 20 | x | x | n | x | x | ο | x | x | x | NH3 | SO2 | |
| 42 | 21 | x | x | n | x | x | x | x | x | x | NH3 | SO2 | |
| 43 | 1 | x | x | o | x | x | o | o | o | o | bw | sw | ja |
| 44 | 2 | x | x | o | x | x | o | x | o | o | bw | sw | ja |
| 45 | 3 | x | x | o | x | x | o | o | x | o | bw | sw | ja |
| 46 | 4 | x | x | o | x | x | o | o | o | x | bw | sw | ja |
| 47 | 5 | x | x | n | x | x | ο | ο | ο | ο | bw | sw | ja |
| 48 | 6 | x | x | n | x | x | ο | x | x | x | bw | sw | ja |
| 49 | 7 | x | x | n | x | x | x | x | x | x | bw | sw | ja |
| 50 | 8 | x | x | o | x | x | o | o | o | o | AHC | sw | ja |
| 51 | 9 | x | x | o | x | x | o | x | o | o | AHC | sw | ja |
| 52 | 10 | x | x | o | x | x | o | o | x | o | AHC | sw | ja |
| 53 | 11 | x | x | o | x | x | o | o | o | x | AHC | sw | ja |
| 54 | 12 | x | x | n | x | x | ο | ο | ο | ο | AHC | sw | ja |
| 55 | 13 | x | x | n | x | x | ο | x | x | x | AHC | sw | ja |
| 56 | 14 | x | x | n | x | x | x | x | x | x | AHC | sw | ja |
| 57 | 15 | x | x | o | x | x | o | o | o | o | NH3 | sw | ja |
| 58 | 16 | x | x | o | x | x | o | x | o | o | NH3 | sw | ja |
| 59 | 17 | x | x | o | x | x | o | o | x | o | NH3 | sw | ja |
| 60 | 18 | x | x | o | x | x | o | o | o | x | NH3 | sw | ja |
| 61 | 19 | x | x | n | x | x | ο | ο | ο | ο | NH3 | sw | ja |
| 62 | 20 | x | x | n | x | x | ο | x | x | x | NH3 | sw | ja |
| 63 | 21 | x | x | n | x | x | x | x | x | x | NH3 | sw | ja |
| 64 | 22 | x | x | o | x | x | o | o | o | o | bw | SO2 | ja |
| 65 | 23 | x | x | o | x | x | o | x | o | o | bw | SO2 | ja |
| 66 | 24 | x | x | o | x | x | o | o | x | o | bw | SO2 | ja |
| 67 | 25 | x | x | o | x | x | o | o | o | x | bw | SO2 | ja |
| | | Ausgeführte Schritte | | | | | | | | | | | |
| 68 | 26 | x | x | n | x | x | ο | ο | ο | ο | bw | SO2 | ja |
| 69 | 27 | x | x | n | x | x | ο | x | x | x | bw | SO2 | ja |
| 70 | 28 | x | x | n | x | x | x | x | x | x | bw | SO2 | ja |
| 71 | 29 | x | x | o | x | x | o | o | o | o | AHC | SO2 | ja |
| 72 | 30 | x | x | x | x | x | o | x | o | o | AHC | SO2 | ja |
| 73 | 31 | x | x | o | x | x | o | o | x | o | AHC | SO2 | ja |
| 74 | 32 | x | x | o | x | x | o | o | o | x | AHC | SO2 | ja |
| 75 | 33 | x | x | n | x | x | ο | ο | ο | ο | AHC | SO2 | ja |
| 76 | 34 | x | x | n | x | x | ο | x | x | x | AHC | SO2 | ja |
| 77 | 35 | x | x | n | x | x | x | x | x | x | AHC | SO2 | ja |
| 78 | 36 | x | x | o | x | x | o | o | o | o | NH3 | SO2 | ja |
| 79 | 37 | x | x | o | x | x | o | x | o | o | NH3 | SO2 | ja |
| 80 | 38 | x | x | o | x | x | o | o | x | o | NH3 | SO2 | ja |
| 81 | 39 | x | x | o | x | x | o | o | o | x | NH3 | SO2 | ja |
| 82 | 40 | x | x | n | x | x | ο | ο | ο | ο | NH3 | SO2 | ja |
| 83 | 41 | x | x | n | x | x | ο | x | x | x | NH3 | SO2 | ja |
| 84 | 42 | x | x | n | x | x | x | x | x | x | NH3 | SO2 | ja |

| | | | | | | | | | | | | | |
|---|---|---|---|---|---|---|---|---|---|---|---|---|---|
| x = wird durchgeführt n = wird nicht durchgeführt o = optional AHC = Ammoniumhydrogencarbonat NH3 = Ammoniak SO2 = Schwefeldioxid bw = Base frei wählbar im Rahmen der Offenbarung sw = Schwefelkomponente frei wählbar im Rahmen der Offenbarung | | | | | | | | | | | | | |

## Patentansprüche

1. Verfahren zur Herstellung eines Vinyllactam-Polymers mit K-Werten von 10 bis 150, das die folgenden Verfahrensschritte a) bis h) umfasst, mit
a) Polymerisation von einem oder mehreren N-Vinyllactamen und optional weiteren Monomeren mittels freier radikalischer Polymerisation mit einem Radikal-bildenden Initiator in einer wässrigen Flüssigkeit, wobei das Polymerisationsverfahren als Batch-Verfahren, als Semi-Batch-Verfahren oder als kontinuierliches Verfahren durchgeführt wird, wobei der Initiatorgehalt 0,01 bis 10 Gewichtsprozent bezogen auf die Menge an Monomeren beträgt,
b) Verwendung wenigstens einer Base um den pH-Wert während der Polymerisation in einem Bereich von 5 bis 11 zu halten;
c) optionale Nachpolymerisation, wobei weiterer Initiator zugegeben werden kann;
d) optionale Reinigung mittels Strippung mit Gas, thermische Destillation und/oder Wasserdampfdestillation;
e) Behandeln des Vinyllactam-Polymeren mit einer Schwefelkomponente, wobei die Schwefelkomponente ausgewählt ist aus der Gruppe bestehend aus schwefliger Säure, Schwefeldioxid und einem oder mehreren Salzen der schwefligen Säure, wobei der pH-Wert einer wasserhaltigen Phase, mit dem das Polymer bei der Behandlung mit der Schwefelkomponente in Kontakt kommt, einen Wert von weniger als 5 aufweist, und In-Kontakt-halten des Polymers mit der wasserhaltigen Phase enthaltend die Schwefelkomponente bei diesem pH-Wert für einen Zeitraum zwischen 10 Minuten und 5 Stunden und anschließend optionale Wiederholung von Schritt d);
f) optionale Zugabe wenigstens einer Base zur Einstellung eines gewünschten pH-Wertes im Bereich von 4 bis 9;
g) optionale Reinigung mittels Filterung;
h) optionales Trocknen zu einem freifließenden Pulver.

2. Verfahren nach Anspruch 1 oder wobei in Schritt f) eine Base ausgewählt aus der Gruppe bestehend aus Ammoniak, Ammonium(hydrogen)carbonat, Triethylamin oder Triethanolamin eingesetzt wird.

3. Verfahren nach einem der Ansprüche 1 bis 2 wobei in Schritt g) ein mechanischer Filter verwendet wird.

4. Verfahren nach einem der Ansprüche 1 bis 3 wobei in Schritt h) ein Sprühtrocknungsverfahren oder ein Kontaktflächentrocknungsverfahren verwendet und ein trockenes Polymerpulver erhalten wird.

5. Verfahren nach einem der Ansprüche 1 bis 4 wobei keine Nachpolymerisation (Schritt c)) ausgeführt wird.

6. Verfahren nach einem der Ansprüche 1 bis 5 wobei in Schritt f) eine Base ausgewählt aus der Gruppe bestehend aus Ammoniak, Ammonium(hydrogen)carbonat, Triethylamin oder Triethanolamin eingesetzt wird, in Schritt g) ein mechanischer Filter und in Schritt h) ein Sprühtrocknungsverfahren oder ein Kontaktflächentrocknungsverfahren verwendet werden.

7. Verfahren nach einem der Ansprüche 1 bis 6 wobei keine Nachpolymerisation (Schritt c)) durchgeführt wird, in Schritt f) eine Base ausgewählt aus der Gruppe bestehend aus Am-" moniak, Ammonium(hydrogen)carbonat, Triethylamin oder Triethanolamin eingesetzt wird, in Schritt g) ein mechanischer Filter und in Schritt h) ein Sprühtrocknungsverfahren oder ein Kontaktflächentrocknungsverfahren verwendet werden, und nach Schritt e) ein weiterer Reinigungsschritt d) durchgeführt wird.

8. Verfahren nach einem der Ansprüche 1 bis 7 wobei als Base in Schritt b) Ammoniumhydrogencarbonat, Ammoniumcarbonat oder Ammoniak verwendet wird.

9. Verfahren nach einem der Ansprüche 1 bis 8 wobei als Schwefelkomponente in Schritt e) Schwefeldioxid in wässriger Lösung verwendet wird.

10. Verfahren nach einem der Ansprüche 1 bis 9 wobei das Verfahren nur aus den Schritten a) bis h) einschließlich der optionalen Wiederholung von Schritt d) nach Schritt e) besteht und keine weiteren Schritte inkludiert sind.

11. Vinyllactam-Polymer erhältlich nach einem Verfahren gemäß einem der Ansprüche 1 bis 10, wobei das Polymer nahezu farblos ist auch bei 30 bis 45 Gewichtsprozent Polymer in wässriger Lösung, einen Restmonomergehalt von maximal 100 ppm aufweist, der Ameisensäuregehalt weniger als 2000 ppm und der "Sulfatasche-Gehalt" höchstens 0,05 Gewichtsprozent beträgt, eine klare wässrige Lösung ohne Trübung ergeben, die wässrigen Lösungen keine messtechnisch relevante Veränderung der Viskosität beim Lagern bei Raumtemperatur (25 °C) und einen geringen bis nahezu nicht vorhandenen, als "typischen Eigengeruch" bezeichneten Geruch aufweisen und kaum staubend sind.

12. Verwendung eines Vinyllactam-Polymers nach Anspruch 11 in kosmetischen oder pharmazeutischen Zubereitungen, Zubereitungen von Agrowirkstoffen, Zubereitungen im Bereich Nahrungsmittel, Tiernahrungsmittel, Nahrungsmittelergänzung oder Tiernahrungsmittelergänzung sowie für technische Anwendungen wie Medizintechnik.

13. Membran zur Reinigung von Flüssigkeiten, insbesondere Dialysemembran, enthaltend ein Vinyllactam-Polymer nach Anspruch 11.

14. Haargel enthaltend Vinyllactam-Polymer nach Anspruch 11.

## Claims

1. A process for producing a vinyllactam polymer having K values of 10 to 150, comprising the following steps a) to h), viz.,
a) polymerizing one or more N-vinyllactams and optionally further monomers via free-radical polymerization with a free-radical initiator in an aqueous liquid, wherein the polymerization process is performed as batch process, as semi-batch process or as continuous process, wherein the initiator content is 0.01 to 10 weight percent based on the amount of monomers,
b) using at least one base to maintain the pH during the polymerization in a range from 5 to 11;
c) optional postpolymerization, wherein further initiator can be added;
d) optional purification by stripping with gas, thermal distillation and/or steam distillation;
e) treating the vinyllactam polymer with a sulfur component selected from the group consisting of sulfurous acid, sulfur dioxide and one or more salts of sulfurous acid, wherein the pH of a water-containing phase with which the polymer comes into contact during the treatment with the sulfur component has a value of less than 5, and keeping the polymer in contact with the water-containing phase comprising the sulfur component at this pH for a period between 10 minutes and 5 hours, and then optional repeat of step d);
f) optionally adding at least one base to set a desired pH in the range from 4 to 9;
g) optional purification by filtering;
h) optional drying to form a free-flowing powder.

2. The process according to claim 1 wherein step f) utilizes a base selected from the group consisting of ammonia, ammonium (hydrogen)carbonate, triethylamine or triethanolamine.

3. The process according to any of claims 1 to 2 wherein step g) utilizes a mechanical filter.

4. The process according to any of claims 1 to 3 wherein step h) utilizes a spray-drying process or a contact-drying process to obtain a dry polymeric powder.

5. The process according to any of claims 1 to 4 wherein no postpolymerization (step c)) is carried out.

6. The process according to any of claims 1 to 5 wherein step f) utilizes a base selected from the group consisting of ammonia, ammonium (hydrogen)carbonate, triethylamine or triethanolamine, step g) utilizes a mechanical filter and step h) utilizes a spray-drying process or a contact-drying process.

7. The process according to any of claims 1 to 6 wherein no postpolymerization (step c)) is carried out, step f) utilizes a base selected from the group consisting of ammonia, ammonium (hydrogen)carbonate, triethylamine or triethanolamine, step g) utilizes a mechanical filter and step h) utilizes a spray-drying process or a contact-drying process, and a further purification step d) is carried out after step e).

8. The process according to any of claims 1 to 7 wherein the base used in step b) is ammonium hydrogencarbonate, ammonium carbonate or ammonia.

9. The process according to any of claims 1 to 8 wherein the sulfur component used in step e) is sulfur dioxide in aqueous solution.

10. The process according to any of claims 1 to 9 wherein the process only consists of steps a) to h) including the optional repeat of step d) after step e) and no further steps are included.

11. A vinyllactam polymer obtainable by a process according to any of claims 1 to 10, wherein the polymer is almost colorless even at 30 to 45 weight percent in aqueous solution, has a residual monomer content of not more than 100 pm, the formic acid content is less than 2000 ppm and the "sulfate ash content" is not more than 0.05 weight percent, giving a clear aqueous solution without turbidity; the aqueous solutions display no measurably relevant change in the viscosity on storage at room temperature (25°C) and have little to almost no "tzpical intrinsic odor" and are scarcely dusting.

12. The use of a vinyllactam polymer according to claim 11 in cosmetic or pharmaceutical preparations, preparations of agroactives, preparations in the sector of food, feed, food supplementation or feed supplementation and also for technical applications such as biomedical engineering.

13. A membrane for purification of liquids, especially a dialysis membrane, comprising a vinyllactam polymer according to claim 11.

14. A hair gel comprising vinyllactam polymer according to claim 11.

## Revendications

1. Procédé de fabrication d'un polymère de vinyllactame ayant des valeurs K de 10 à 150, qui comprend les étapes de procédé a) à h) suivantes :
a) la polymérisation d'un ou de plusieurs N-vinyllactames et éventuellement de monomères supplémentaires par polymérisation radicalaire libre avec un initiateur formant des radicaux dans un liquide aqueux, le procédé de polymérisation étant réalisé sous la forme d'un procédé discontinu, sous la forme d'un procédé semi-discontinu ou sous la forme d'un procédé continu, la teneur en initiateur étant de 0,01 à 10 pour cent en poids, par rapport à la quantité de monomères,
b) l'utilisation d'au moins une base afin de maintenir le pH pendant la polymérisation dans une plage allant de 5 à 11 ;
c) éventuellement la polymérisation secondaire, davantage d'initiateur pouvant être ajouté ;
d) éventuellement la purification par extraction avec un gaz, distillation thermique et/ou distillation à la vapeur d'eau ;
e) le traitement du polymère de vinyllactame avec un composant soufré, le composant soufré étant choisi dans le groupe constitué par l'acide sulfurique, le dioxyde de soufre et un ou plusieurs sels de l'acide sulfurique, le pH d'une phase aqueuse avec laquelle le polymère rentre en contact lors du traitement avec le composant soufré présentant une valeur de moins de 5, et la mise en contact du polymère avec la phase aqueuse contenant le composant soufré à ce pH pendant une durée comprise entre 10 minutes et 5 heures, puis éventuellement la répétition de l'étape d) ;
f) éventuellement l'ajout d'au moins une base pour l'ajustement d'un pH souhaité dans la plage allant de 4 à 9 ;
g) éventuellement la purification par filtration ;
h) éventuellement le séchage en une poudre fluide.

2. Procédé selon la revendication 1, dans lequel une base choisie dans le groupe constitué par l'ammoniac, l'(hydrogéno) carbonate d'ammonium, la triéthylamine ou la triéthanolamine est utilisée à l'étape f).

3. Procédé selon l'une quelconque des revendications 1 à 2, dans lequel un filtre mécanique est utilisé à l'étape g).

4. Procédé selon l'une quelconque des revendications 1 à 3, dans lequel un procédé de séchage par pulvérisation ou un procédé de séchage par surfaces de contact est utilisé à l'étape h), et une poudre polymère sèche est obtenue.

5. Procédé selon l'une quelconque des revendications 1 à 4, dans lequel aucune polymérisation secondaire (étape c)) n'est réalisée.

6. Procédé selon l'une quelconque des revendications 1 à 5, dans lequel une base choisie dans le groupe constitué par l'ammoniac, l'(hydrogéno)carbonate d'ammonium, la triéthylamine ou la triéthanolamine est utilisée à l'étape f), un filtre mécanique est utilisé à l'étape g), et un procédé de séchage par pulvérisation ou un procédé de séchage par surfaces de contact est utilisé à l'étape h).

7. Procédé selon l'une quelconque des revendications 1 à 6, dans lequel aucune polymérisation secondaire (étape c)) n'est réalisée, une base choisie dans le groupe constitué par l'ammoniac, l'(hydrogéno) carbonate d'ammonium, la triéthylamine ou la triéthanolamine est utilisée à l'étape f), un filtre mécanique est utilisé à l'étape g), et un procédé de séchage par pulvérisation ou un procédé de séchage par surfaces de contact est utilisé à l'étape h), et une étape de purification d) supplémentaire est réalisée après l'étape e).

8. Procédé selon l'une quelconque des revendications 1 à 7, dans lequel l'hydrogénocarbonate d'ammonium, le carbonate d'ammonium ou l'ammoniac est utilisé en tant que base à l'étape b).

9. Procédé selon l'une quelconque des revendications 1 à 8, dans lequel du dioxyde de soufre en solution aqueuse est utilisé en tant que composant soufré à l'étape e).

10. Procédé selon l'une quelconque des revendications 1 à 9, dans lequel le procédé n'est constitué que par les étapes a) à h), y compris la répétition éventuelle de l'étape d) après l'étape e), et aucune étape supplémentaire n'est incluse.

11. Polymère de vinyllactame pouvant être obtenu par un procédé selon l'une quelconque des revendications 1 à 10, le polymère étant pratiquement incolore, même à 30 à 45 pour cent en poids de polymère dans une solution aqueuse, présentant une teneur résiduelle en monomères d'au plus 100 ppm, la teneur en acide formique étant inférieure à 2 000 ppm et la « teneur en cendres sulfatées » étant d'au plus 0,05 pour cent en poids, permettant d'obtenir une solution aqueuse transparente sans trouble, les solutions aqueuses ne présentant pas de modification de la viscosité pertinente en termes de mesures lors d'un stockage à température ambiante (25 °C), et présentant une odeur dénommée « odeur propre type» faible à pratiquement non présente, et formant peu de poussières.

12. Utilisation d'un polymère de vinyllactame selon la revendication 11 dans des préparations cosmétiques ou pharmaceutiques, des préparations d'agents actifs agrochimiques, des préparations dans le domaine des produits alimentaires, des aliments pour animaux, des compléments alimentaires ou des compléments alimentaires pour animaux, ainsi que pour des applications techniques telles que les techniques médicales.

13. Membrane pour la purification de liquides, notamment membrane de dialyse, contenant un polymère de vinyllactame selon la revendication 11.

14. Gel pour les cheveux contenant un polymère de vinyllactame selon la revendication 11.
